# EUROPEAN PATENT SPECIFICATION

(11) **EP 3 183 583 B1**
(45) Date of publication and mention of the grant of the patent: **13.07.2022**
(21) Application number: 15757188.6
(22) Date of filing: 20.08.2015
(51) Int. Cl.: G01N 33/68

(54) **METHOD FOR SELECTING A TRANSPLANTATION DONOR OR DONOR MATERIAL USING LOW OR INTERMEDIATE HLA TYPING INFORMATION**
VERFAHREN ZUR AUSWAHL EINES TRANSPLANTATIONSSPENDERS ODER SPENDERMATERIALS MITINFORMATIONEN ÜBER HLA-TYPISIERUNG MIT NIEDRIGER ODER MITTLERER AUFLÖSUNG
MÉTHODE DE SÉLECTION D'UNE MATIÈRE DE TYPE DONNEUR OU D'UN DONNEUR DE TRANSPLANTATION À L'AIDE D'INFORMATIONS DE TYPAGE HLA FAIBLE OU INTERMÉDIAIRE

(30) Priority: 20.08.2014 EP 14181626; 25.08.2014 US 201462041295 P
(43) Date of publication of application: 28.06.2017
(73) Proprietor: PIRCHE AG, 10785 Berlin (DE)
(72) Inventor: MCCLOSKEY, Daniel, West Wickham Kent BR4 0DW (GB)
(74) Representative: Hertin und Partner Rechts- und Patentanwälte PartG mbB
(86) International application number: PCT/EP2015/069163
(87) International publication number: WO 2016/026934

(56) References cited:
- WO-A1-2015/169597
- KIRSTEN GENEUGELIJK ET AL: "Predicting Alloreactivity in Transplantation", JOURNAL OF IMMUNOLOGY RESEARCH, vol. 32, no. 9, 1 January 2014 (2014-01-01), pages 2510-12, XP055201699, ISSN: 2314-8861, DOI: 10.1146/annurev.immunol.23.021704.115658

## Description

The invention relates to the field of transplantation and implantation medicine, in particular the selection of a transplantation donor or donor material for allogeneic transplantation based on molecular HLA typing and computer-implemented methods. The invention relates to a method for selecting a mismatched transplantation donor for providing transplantation material or selecting a mismatched allele group of a transplantation donor, wherein two-field specific HLA protein typing is unavailable for said donor(s). The method comprises determining in a computer-implemented method the number of predicted indirectly recognized HLA epitopes (PIRCHES) for multiple specific HLA proteins of the donor allele group of the HLA locus that is mismatched or potentially mismatched between said donor and said recipient (mismatched donor allele group). The candidate transplantation donors or one or more mismatched allele groups of a transplantation donor are then selected according to the PIRCHE values (scores). The invention also relates to computer-software and systems for carrying out said method.

### BACKGROUD OF THE INVENTION

Transplantation of allogeneic cells, tissues and organs is an evolving therapy that has become an increasingly attractive therapeutic option. The number of patients receiving transplants from unrelated donors is expected to double in the near future. Alloreactivity after transplantation has a major impact on clinical outcome, with pathological as well as beneficial effects. HLA mismatches are known to induce an immune reaction after transplantation, however the factors involved in predicting risk of unwanted immune reaction are not well understood.

Hematopoietic Stem Cell Transplantation (HSCT) is one example of a quickly growing therapeutic option. The major limiting factor of HSCT remains graft-versus-host disease (GVHD), and since the number of patients receiving HSCT is expected to increase, the provision of novel approaches to prevent GVHD must be accelerated.

The risk of acute GVHD (aGVHD) is dependent on the level of matching for the HLA-A, -B, -C, - DRB1, -DQB1 and possibly DPB1 alleles, with an optimal match being a full match for five loci (HLA-A, -B, -C, -,DRB1, -DQB1, a 10/10 match). However, fully matched-unrelated donors are not available for all patients; in 40% of the situations, a single HLA-mismatched donor (a 9/10 match) or a donor who is potentially mismatched, who has only been typed at low or intermediate resolution, is the best available alternative. In these situations, definition of the best-permissible mismatch may help prevent GVHD and, subsequently, inferior outcome. When a completely matched donor is not available, a clinician often has to face the difficult decision to choose the best donor out of the mismatched donors (i.e. the one that carries the lowest GVHD risk).

Until now, determining which donor is most suitable relies on a laborious assay that requires up to 14 days of lab-work. Functionally, better-permissible mismatches are determined with cytotoxic T-lymphocyte precursor frequency (CTLpf) assays. However, the CTLpf assay is laborious, delays time to transplantation, and is therefore not used in most transplant centres.

To find an alternative for the CTLpf assay, multiple, so far unsuccessful, attempts have been undertaken to predict non-permissible mismatches using prediction programs, such as HLAMatchmaker. HLAMatchmaker determines potential epitopes for antibodies and has proven its validity for solid-organ transplantation. HLAMatchmaker considers differences in amino-acid triplets as epitopes on HLA. Although antibodies potentially play a role in the development of GVHD, predictions based on HLAMatchmaker are not correlated to alloreactivity.

Similar difficulties exist with respect to identifying acceptable low-risk donor material for organ transplantation, for example kidney transplantation. HLA mismatches between donor and recipient exist in approximately 85% of cadaveric kidney transplantations. Evidently, these HLA mismatches frequently lead to production of HLA-specific antibodies, which shorten graft survival and reduce re-transplantation options. In order to prevent antibody formation against HLA, the optimal kidney grafts are either HLA identical to the recipient, or express acceptable HLA mismatches which do not induce antibody formation (host versus graft response; HVGD).

In light of previous techniques there exists a need for more reliable methods for predicting whether donor material for a transplantation, which is HLA mismatched, is at increased risk of leading to a failed transplantation, for example development of GVHD or HVGD, and/or an increase in mortality.

Recent findings have shown that indirect recognition of a mismatched-HLA antigen may lead to T cell-related alloreactivity. During indirect HLA recognition, T cells recognize peptides derived from polymorphic HLA antigens presented by a shared (matched) HLA molecule. Peptides derived from mismatched-HLA molecules are frequently presented by HLA. These indirectly recognizable HLA epitopes have been associated with both acute and chronic graft failure in solid organ transplantation. T cells that indirectly recognize HLA-mismatches in the context of self-HLA may therefore play an important role in clinical alloreactivity.

To facilitate the identification of indirectly recognizable HLA epitopes, a predictive tool has been developed to assess the risk of an unwanted T cell alloreactivity on the basis of predicted proteasome cleavage of HLA molecules and subsequent HLA presentation of the HLA peptide. The corresponding method is disclosed in WO 2014/072467 A1 and Otten et al (Hum Immunol. 2013 Mar;74(3):290-6). The methods described therein designate the HLA-derived epitopes that are predicted to be presented as Predicted Indirectly ReCognizable HLA Epitopes (PIRCHES). Based on this method, the numbers of predicted PIRCHES can be assessed and correlated to risk of an unwanted immune reaction. The method described therein enables the prediction of permissible and non-permissible HLA mismatches prior to HSCT and other cell or organ transplants or implants.

WO 2015/169597 A1 discloses a method, system and data structure for carrying out a computer implemented method for determining the numbers of predicted indirectly recognized HLA epitopes (PIRCHES) between one or more donors and one or more recipients of transplantation material. The method, system and data structure described allow the identification of permissible mismatches and therefore relatively safe transplantation material by analysing via a computer implemented method HLA-derived peptides of patient and donor. Geneugelijk K et al (Journal of Immunological Research, (1 January 2014) vol. 32, no. 9, pages 2510-12) present a review of the methods available for predicting alloreactivity in transplantation and address the prediction of indirect T-Cell recognition using the PIRCHES method. There is however no teaching or indication in the prior art of applying the PIRCHE technology to situations where 2-field specific HLA typing data is unavailable or incomplete.

Despite the developments in the prior art regarding the use of PIRCHES for predicting T cell mediated alloreactivity, the methods of the art are limited by the requirement of "high resolution" HLA typing information (such as specific HLA protein sequence typing) for both donor and recipient. Similar and less effective approaches, such as HLAMatchmaker, are also limited to the use of high resolution typing information.

At present, only a limited number of donors and/or recipients have been typed at high resolution. Donor databases, for example those cataloguing stem cells available for allogeneic transplantation and all solid organ donors, may only exhibit a small number of samples that have been typed at high resolution, therefore significantly limiting the practical implication of the PIRCHES method as previously described.

So-called "low resolution" HLA typing relates to identification of the HLA allele group at any given locus, without providing information on which particular protein sequence is in fact present. Within any given allele group a potentially large number of variant HLA protein sequences may be present. Low resolution typing relates to providing information at the level of the digits comprising the first field in standard HLA nomenclature. An examples is HLA-A*01. All specific proteins in the A*01 allele group will share some similarity in sequence, although important differences in sequence with respect to a potential alloreactivity may occur between the specific proteins falling within the A*01 allele group.

The National Marrow Donor Program (NMDP) operates a registry of volunteer hematopoietic cell donors and umbilical cord blood units in the United States. The NMDP uses allele codes to facilitate the reporting of HLA alleles and to characterize donor material stored for transplant. The NMDP allele codes are however in some cases generic, wherein one code may encompass a number of possible HLA sequences (alleles) at any given HLA locus. Although the donor material may be HLA typed, a number of NMDP codes do not provide information on which specific protein is in fact present at any given HLA locus. The HLA typing of these particular samples is therefore considered incomplete (or may be termed "intermediate resolution" HLA typing).

A large number of donor samples stored for transplantation are at present only typed at either "low" or "intermediate" resolution. When no completely matched donor material is evident, some of the mismatched donor material may also be suitable for transplantation. However, clinicians searching for matched donor material face a difficult task in selecting "low" or "intermediate" resolution typed material for transplantation, or for further high resolution typing before transplantation. Determining which donor is most likely to provide acceptable allogeneic material, or which mismatched allele group is most likely to be associated with a low risk of an adverse immune reaction, remains a challenge for clinicians when faced with "low" or "intermediate" resolution typed donor information.

Further methods are required in the fields of cell and organ transplantation and implantation for the identification of suitable donor material, in particular from mismatched or potentially mismatched donors or donor samples, for whom only "low" or "intermediate" resolution typing information is available.

### SUMMARY OF THE INVENTION

In light of the prior art the technical problem underlying the present invention is the provision of means for identifying transplantation donors or donor material, for which incomplete, or only low or intermediate resolution, typing information is available. Another problem underlying the invention is the provision of means for selecting donor material, for which incomplete, or only low or intermediate resolution, typing information is available, which is suitable for allogeneic transplantation and is associated with a low risk of adverse reaction.

This problem is solved by the features of the independent claims. Preferred embodiments of the present invention are provided by the dependent claims.

The invention is set out in the appended set of claims.

The invention therefore relates to a method for selecting a donor for providing transplantation material (or selecting a mismatched allele group of a transplantation donor), comprising:
a. Providing an electronic representation of HLA-typing data from a transplantation recipient and one or more candidate transplantation donors, wherein two-field specific HLA protein typing is unavailable for said donor(s) and/or recipient,
b. Determining in a computer-implemented method the number of predicted indirectly recognized HLA epitopes (PIRCHES) (hereinafter "PIRCHE value") for multiple specific HLA proteins of the donor allele group of the HLA locus that is mismatched or potentially mismatched between said donor and said recipient (hereinafter "mismatched donor allele group"); and
c. Selecting or rejecting one or more of said candidate transplantation donors in a computer-implemented method according to the PIRCHE values determined in step b).

In one embodiment, the transplantation donor to be selected is associated with a reduced risk of an unwanted immune response (such as a T cell mediated alloreactivity) against human leukocyte antigens (HLA) after transplantation. In one embodiment the transplantation donor to be selected is associated with an increased likelihood of exhibiting an acceptable number of PIRCHES for transplantation in the recipient.

The invention therefore relates to a method for selecting mismatched or potentially mismatched candidate donors, for whom only low or intermediate resolution HLA typing information is available, for providing transplantation material or for further high resolution HLA typing, wherein said selection incorporates the determination of Predicted Indirectly ReCognizable HLA Epitopes (PIRCHES) between one or more candidate donors and recipients of transplantation material. The invention utilises the determination of PIRCHES using multiple potential specific HLA proteins of a mismatched donor allele group in order to increase the likelihood of selecting a mismatched candidate donor with a low number of PIRCHES and therefore an acceptable mismatch for the transplantation.

The embodiments of the invention regarding selection of a transplantation donor relate to a method wherein one or more transplantation donors are assessed for their likelihood of providing an acceptable mismatch for allogeneic transplantation or wherein one or more transplantation recipients are assessed for their likelihood of providing an acceptable mismatch for allogeneic transplantation.

In one embodiment, multiple donors typed at low or intermediate resolution are analysed by the method incorporating the PIRCHE analysis as disclosed herein, wherein multiple (preferably essentially all known) possible specific HLA proteins of the mismatched donor allele are analysed for their PIRCHE value. Typically, the donor is then selected having the mismatched allele group that shows the lowest PIRCHE values, for example the lowest Median PIRCHE values.

The invention also relates to a method for selecting a mismatched allele group of a transplantation donor, comprising:
a. Providing an electronic representation of HLA-typing data from a transplantation recipient and one or more candidate transplantation donors, wherein two-field specific HLA protein typing is unavailable for said donor(s) and/or recipient,
b. Determining in a computer-implemented method the PIRCHE value for multiple specific HLA proteins of multiple allele groups of a mismatched allele; and
c. Selecting or rejecting one or more mismatched allele groups of a transplantation donor in a computer-implemented method according to the PIRCHE values determined in step b).

In one embodiment, the mismatched allele group of the transplantation donor to be selected is associated with a reduced risk of an unwanted immune response (such as a T cell mediated alloreactivity) against human leukocyte antigens (HLA) after transplantation. In one embodiment the mismatched allele group of the transplantation donor to be selected exhibits a relatively lower number of PIRCHES compared to other mismatched allele groups.

In the embodiments of the invention regarding selection of a mismatched allele group, a user of the method is able to identify on the basis of the recipient HLA typing information (preferably available in high resolution, or more preferably in two-field specific HLA protein resolution) which allele group mismatch would be most likely to provide a low PIRCHE value and therefore be a suitable mismatch for allogeneic transplantation. In a preferred embodiment, the user of the method may be able to subsequently look in databases or registries of donor material for a mismatched sample, which comprises a mismatch in the particular identified allele group, as the method enables selection of a mismatched allele group that is (for the particular assessed recipient) associated with increased likelihood of providing a donor (when typed at high resolution) with an acceptable mismatch.

According to the present invention, the transplantation recipient and candidate transplantation donor(s) may be virtual representations of said recipients or donors. The method relates to computer-implemented methods that enable the processing of HLA typing information of real patients or real donor material, for which the HLA typing data has been stored and can be processed electronically.

According to the present invention the term "providing a transplantation recipient" or "providing a candidate transplantation donor" may relate to providing data, such as computer data, for example HLA typing data, or an in silico representation of data, on a recipient and/or donor. The term "providing" may also relate to selection of a donor and/or recipient, or identifying a donor and/or recipient, for example in a computer or simulated method, or in a method involving particular individuals as donors and/or recipients as real subjects. The method may also encompass a method wherein only some steps are carried out as a computer implemented method and the remaining steps with real subjects or materials. The method may also comprise the computer-implemented analysis of PIRCHES and the provision of real transplantation material.

The invention may also encompass the sourcing, storage, delivery and use of real transplantation material. As an example, the computer implemented steps of the method may be combined with a connection to a database of stored transplantation material, the method as described herein may also encompass identification of particular deposited samples from donors, ordering said samples and/or delivery of samples to the clinic responsible for the transplantation.

In one embodiment, the method as described herein is characterised in that the candidate transplantation donor is selected for further typing, preferably specific HLA protein typing (high resolution typing), at one or more HLA loci before providing transplantation material for a biological transplantation procedure. Through the method described herein, clinicians may identify those donors who have a relatively high likelihood of exhibiting a low PIRCHES value.

The present invention therefore saves significant time and financial cost by the pre-selection of candidate donors with mismatches, which are more likely than other donors to be acceptable mismatches for allogeneic transplantation.

The present invention relates to a determination of the PIRCHES value for multiple specific HLA proteins of the mismatched donor allele group. In a preferred embodiment, the method incorporates essentially all, or every known, or the most likely possible protein variants (for example based on allele frequency tables or user defined tables) of the allele group, in order to effectively determine whether a particular donor or allele group indeed is associated with a relatively low PIRCHE value and therefore present itself as a preferred selection. Because those skilled in the art are able to make a pre-selection of possible protein variants within any given allele group, either on the basis of the reliability of the stored data or on population frequencies of certain alleles, the method is not limited to analysis of all known protein sequences of an allele group. In one embodiment the method will encompass the analysis of essentially all known (or relevant) protein sequences within the allele group, in order to provide more definitive results.

The present invention may also relate to the determination of the PIRCHE value for multiple specific HLA proteins of multiple allele groups of a mismatched allele. Because those skilled in the art are able to make a pre-selection of possible allele groups at any given locus, either on the basis of the reliability of the stored data or on population frequencies of certain alleles, the method is not limited to analysis of all known allele groups at any given locus. In one embodiment the method will encompass the analysis of essentially all known (or relevant) protein sequences within the allele group, in order to provide more definitive results.

According to the present invention the term mismatched donor allele group is used to describe the donor allele group of the HLA locus that is mismatched or potentially mismatched between said donor and said recipient. The term mismatched donor allele group therefore relates to mismatched or potentially mismatched allele groups. If a mismatch is evident, PIRCHE is then carried out on each of the specific protein variants of that allele group. However, if the HLA typing information provided for a candidate donor is incomplete, as is often the case, the donor may in fact be matched, but is characterised as a mismatch due to the missing information. Such candidate donors may also be assessed by the present invention.

In a preferred embodiment, the method as described herein is characterised in that at least one HLA mismatch (at any HLA loci) is present between said donor(s) and said recipient at the allele group level.

In a preferred embodiment, the method as described herein is characterised in that specific HLA protein typing information (otherwise known as high resolution typing information), preferably two-field specific HLA protein typing information, is available for said recipient.

Although high resolution typing information for the recipient is preferred, the method may be adapted to be carried out for recipients who also do not have complete information with respect to their HLA typing. In this embodiment, the method as described herein is characterised in that two-field specific HLA protein typing (specific HLA protein typing information; high resolution typing) is unavailable and HLA allele group typing information (any low or intermediate resolution typing) is available for said recipient.

The method as described herein is preferably characterised in that said transplantation donor or mismatched allele group of a transplantation donor is associated with reduced risk of an unwanted immune response against human leukocyte antigens (HLA) after transplantation.

In a preferred embodiment, the method as described herein is characterised in that the likelihood (risk) of an unwanted immune response against human leukocyte antigens (HLA) in the recipient after transplantation is lower for transplantation material obtained from donors with a relatively greater proportion of specific-HLA proteins of the mismatched donor allele group that exhibit relatively lower PIRCHE values compared to other candidate donors.

In a preferred embodiment, the method as described herein is characterised in that the PIRCHE value is the sum of PIRCHE-I and PIRCHE-II values. The method may however be carried out for either PIRCHE I or PIRCHE II values and either or both of the said values used to assess donor and/or recipient suitability. Such an analysis may be carried out according to the particular biological background relevant to the recipient. Such specific scenarios, for example for which kind of transplantations which PIRCHE values should be assessed, are provided in WO 2014/072467 A1

In one embodiment, the method as described herein is characterised in that the likelihood (risk) of an unwanted immune response against human leukocyte antigens (HLA) in the recipient after transplantation is lower for transplantation material obtained from donors with a relatively greater proportion of specific HLA proteins of the mismatched donor allele group that exhibit PIRCHE values less than or equal to a pre-set value (defined by the user), compared to other candidate donors.

In one embodiment, the method as described herein comprises determining the distribution of PIRCHE values (via appropriate statistical methods) of multiple specific HLA proteins of the mismatched donor allele group, wherein a candidate transplantation donor is selected for providing transplantation material when a relatively greater proportion of specific-HLA proteins of the mismatched donor allele group exhibit relatively lower PIRCHE values compared to other candidate donors.

In one embodiment, the method as described herein comprises determining the median PIRCHE value of multiple specific HLA proteins of the mismatched donor allele group, wherein a candidate transplantation donor is selected for providing transplantation material when the median PIRCHE value is less than or equal to a pre-set value, preferably wherein a candidate transplantation donor is selected for providing transplantation material when the median PIRCHE value is less than or equal to 10, more preferably less than or equal to 5.

In one embodiment, the method as described herein comprises determining the median PIRCHE value and standard deviation of the specific HLA proteins of the mismatched donor allele group.

In one embodiment, the method as described herein comprises determining the percentage of the specific HLA proteins of the mismatched donor allele group that have a PIRCHE value less than or equal to a pre-set value, wherein a candidate transplantation donor is selected for providing transplantation material when greater than the pre-set percentage, preferably 50%, of the specific HLA proteins of the mismatched donor allele group have a PIRCHE value less than or equal to a pre-set value, preferably less than or equal to 10, more preferably less than or equal to 5.

There has been no suggestion in the art that a particular threshold exists for an "acceptable number" of such peptides. This aspect of the invention, in addition to the further embodiments that are defined by specific values indicative of donors to be selected or not selected, enable complex biological phenomena to be assessed in the form of a simple "yes-no" read-out, thereby providing feedback to the end-user of the method whether any given donor material is to be selected. This subsequently enables a completely automated approach towards interrogating databanks comprising HLA typing data, and a method in which a clear indication is provided whether material from a mismatch donor may be relatively safe for application and/or should be selected for further HLA typing.

In one embodiment, the method as described herein is characterised in that the candidate transplantation donor is selected for providing transplantation material for a biological transplantation procedure, wherein the biological transplantation is preferably a cell transplantation, such as a stem cell transplantation, for example of hematopoietic stem cells (HSC), preferably HSC obtained from cord blood, or related/ unrelated HSC donors. Although the analysis provided herein is primarily intended for the pre-selection of candidate donors for subsequent HLA typing, the donors selected by the method may be used directly for sourcing donor material.

The term candidate donor may also refer to donor material suitable for a transplantation. The donor material may have already been isolated and stored in an appropriate database or registry and the HLA typing information processed by the present method.

In one embodiment the method as described herein may be characterised in that providing a candidate transplantation donor(s) and transplantation recipient comprises electronic (in silico) representation and/or processing of the HLA typing information available for said donor and said recipient in a computer-implemented method.

In one embodiment the method as described herein may be characterised in that determining the number of predicted indirectly recognized HLA epitopes (PIRCHES) comprises determining the number of predicted recipient- or donor-specific HLA-derived peptides from the mismatched recipient-HLA allele that are predicted to be presented by a shared (matched) HLA molecule in a computer-implemented method.

In one embodiment the method as described herein may be characterised in that the sequences of specific HLA proteins of any given mismatched donor allele group are provided, obtained and/or processed as an electronic (in silico) representation of possible (known to exist in the human population) HLA protein sequences of any given HLA locus, preferably stored in a databank comprising multiple, preferably essentially all, known HLA allele sequences.

In one embodiment the method as described herein may be characterised in that selecting or rejecting one or more of said candidate transplantation donors according to the PIRCHE values is carried out in a, preferably automated, computer-implemented method.

In one embodiment the method as described herein may be characterised in that candidate donors are electronically (in silico) generated to represent every possible mismatched specific HLA protein combination to the transplantation recipient in a computer-implemented method.

In one embodiment, the method as described herein comprises determining the PIRCHE value for multiple specific HLA protein of multiple allele groups of a mismatched allele for multiple mismatched HLA loci, thereby identifying a mismatched HLA locus associated with relatively low PIRCHE values compared to other mismatched HLA loci.

In one embodiment, the method as described herein comprises processing information representing the frequency of any given specific HLA protein and/or HLA allele group (haplotype or genotype) in any one or more human populations, such as defined by age, gender and/or ethnicity, during selecting a donor or mismatched allele group.

For example, the frequency that any given allele occurs in any given human population can be determined. Public databases are available containing such information, for example the Allele Frequency Net Database (AFND) provides a central source for the storage of allele frequencies in various human populations from different polymorphic areas in the Human Genome (Gonzalez-Galarza et al., Nucleic Acid Research 2011, 39, D913-D919). Through this approach a skilled person, or user of the method, may adjust the allele groups of HLA proteins of any given allele groups within the analysis.

The present invention therefore relates to a method for reducing the risk of an unwanted immune response against human leukocyte antigens (HLA) after transplantation in a recipient and/or for increasing the likelihood of obtaining a mismatched transplantation donor associated with a reduced risk of inducing said unwanted immune response comprising carrying out the method as described herein.

The invention also relates to the use of a method for predicting an immune response against human leukocyte antigens (HLA) after transplantation, said method comprising determination of the number of predicted indirectly recognized HLA epitopes (PIRCHES), wherein said PIRCHES are recipient- or donor-specific HLA-derived peptides from the mismatched recipient-HLA allele and are predicted to be presented by a shared (matched) HLA molecule, for the selection of a transplantation donor for providing transplantation material, wherein HLA protein-specific typing information (high resolution typing) is unavailable for said donor.

The invention also relates to a system for selecting a transplantation donor for providing transplantation material, said system comprising one or more computing devices, data storage devices and/or software as system components, wherein said components may be connected in close proximity to one another or via a data connection, for example over the internet, and are configured to interact with one or more of said components and/or to carry out the computer-implemented steps of the method described herein. The system may comprise computing devices, data storage devices and/or appropriate software, for example individual software modules, which interact with each other to carry out the method as described herein.

The method therefore provides means for pre-transplantation prediction of the risk or likelihood of occurrence of an unwanted immune response against human leukocyte antigens (HLA), which could occur after transplantation. The HLA-typing of the method has typically been carried out in advance and the data regarding HLA-type subsequently analysed via the method of the present invention.

According to the present invention, the PIRCHES are typically recipient-specific peptides in cases of HSCT and GVHD, GVL and reduced survival, whereas the PIRCHES may be donor-specific in cases of graft rejection, after organ or tissue transplantation or implantation.

The present invention represents a technical utilisation of the relationship between risk of alloreactivity and increased numbers of mismatched HLA-derived peptides presented by shared-HLA molecules. These numbers can be determined preferably *in silico* and can be used as a predictive marker with respect to the development of alloreactivity, for example GVHD.

The present invention provides a preferably computer implemented method that determines which donor is suited for transplantation when a completely matched donor is not available, without the need for laborious compatibility assays. The present invention for example is applicable to multiple transplant settings, such as stem cells, cord blood cells or solid organ transplantation, amongst others. Essentially any transplantation or implantation procedure, in which HLA-matching plays a role in determining alloreactivity or tissue rejection, is encompassed by the present invention.

Considering the enormous health and emotional cost to patients having suffered from unwanted immune responses after transplantation, in addition to relatively high costs for carrying out high resolution typing, methods for the prediction of safely transplantable or implantable material and for the prediction of which mismatched or potentially mismatched donors are associated with a lower risk of an unwanted immune reaction, are of paramount importance to the medical community. The method as described herein enables reduction of risk upstream of surgery (or treatment) and upstream of any high resolution typing that may be required, thereby avoiding substantial health and financial cost to patients, medical practitioners and institutions, respectively.

The determination of PIRCHES values as used herein relates to a method for the prediction of an immune response against human leukocyte antigens (HLA) associated with, preferably induced by, HLA-mismatches between donor and recipient after transplantation, wherein HLA-typing for the donor and recipient is conducted using high resolution sequence specific HLA typing information (in this case theoretical sequences of known HLA alleles for mismatched incompletely typed donors). The number of predicted indirectly recognized HLA epitopes (PIRCHES) is identified using computer-implemented methods by determining the presentation and/or binding of peptides derived from mismatched recipient and/or donor HLA alleles, whereby the number of PIRCHES correlates with the likelihood of said immune response.

In one embodiment the present invention relates to a method as described herein, wherein transplantation donor or transplantation material is suitable for haematopoietic stem-cell transplantation (HSCT).

In one embodiment the present invention relates to a method as described herein, wherein transplantation donor or transplantation material is suitable for hematopoietic stem cells derived from cord blood or from adult donors for transplantation.

In one embodiment the present invention relates to a method as described herein, wherein transplantation donor or transplantation material is suitable for any solid organ or tissue transplantation.

In one embodiment the present invention relates to a method as described herein, wherein the risk of an unwanted alloreactivity is to be reduced. In one embodiment the present invention relates to a method as described herein, wherein the risk of an immune response is to be increased, such as for an anti-leukemic alloreactivity.

In some embodiments, the determination of PIRCHE values as described herein is characterised in that the likelihood of an immune response is increased with an increase in the number of PIRCHES. Depending on the cut-off values used in the determination of PIRCHE value, in general, patients with high numbers (>2) of peptides presentable by HLA class I compared to patients with low numbers (≤2), developed aGVHD significantly earlier. Patients suffering from extensive cGVHD displayed a trend for higher numbers of peptides presentable by HLA class I and II combined and by HLA class I and II separately, as compared to those with no or limited cGVHD. Extensive cGVHD developed earlier in the patients that were predicted to present high numbers of peptides on HLA class I and II combined compared to low numbers. Patients with both low PIRCHE-I and PIRCHE-II values typically show the lowest risk estimates, similar to 10/10 matched transplantations. Especially donor-recipient combinations in the low PIRCHE-I group are favourable: these combinations have a significantly increased probability of survival and disease-free survival, and show a decreased risk of acute and chronic GvHD compared to patients in the higher PIRCHE-I groups. On the basis of these correlations, through the present method, mismatched donors may be selected that provide essentially the same low risk of unwanted immune responses as HLA-matched donors.

The computer implementation of the invention enables an efficient, fast and reliable method for identification of potentially permissible donor material for allogeneic transplantation. The data processed by the software can be handled in a completely or partially automatic manner, thereby enabling in a preferred embodiment an automated computer-implemented method. Data regarding the HLA typing of donor and recipient, in addition to the number of PIRCHES determined for any donor-recipient pair, can be stored electronically and maintained in appropriate databases. The invention therefore also relates to computer software capable of carrying out the method as described herein.

The system preferably comprises a database with information on all published HLA alleles. The database may be updated as new HLA allele sequences are published. Computer software, preferably based on PERL, but which could also be based on other computing languages, can be used for generating and/or updating the databases, in addition to calling the respective programmes required for assessing the number of PIRCHES.

In one embodiment the invention may comprise a system of databanks or databases of a cord blood bank, whereby each sample is tested for HLA-type, and the information stored electronically. The system may also comprise a connection between an additional computing device, for example a device of a clinician, transplant centre, or hospital, in which the HLA-type data for the recipient is stored. Through a connection between the two databases, for example over the internet, the method of the invention can be carried out using appropriate software. HLA-types of multiple potential donor samples and the patient may be compared and the number of PIRCHE for any given donor-recipient pair determined. In light of the analysis based on the method described herein, a clinically relevant prediction can be made whether any given donor material, for example those samples stored in a cord blood bank or other cell or tissue bank, is suitable for transplantation. The invention also relates to a software suitable for carrying out the method described herein.

Upon identification of HLA-matched material the transplantation could be conducted. In cases where material is identified in which HLA-mismatches between the donor and recipient are evident, the permissibility of the mismatches can be ascertained by application of the method or system of the present invention to any given or all possible donor-recipient pairs. Appropriate software, capable of carrying out the determination of the number of PIRCHES, may be applied for any given donor-recipient pair and, on the basis of the number of PIRCHES, the permissibility of the HLA-mismatch assessed, preferably automatically.

In one embodiment the determination of PIRCHES as described herein relates to a method, wherein said recipient- or donor-specific HLA-derived peptides from the mismatched recipient-HLA allele are identified by a computer-implemented method for identifying the cleavage sites (endopeptidase and/or protease sites) of the human proteasome. A preferred embodiment of this feature of the invention relates to the use of the software NetChop, or alternative software as described herein, which is capable of determining proteasome cleavage of peptide sequences.

In one embodiment the determination of PIRCHES as described herein relates to a method, wherein said presentation of peptides by a shared (matched) HLA molecule is determined by a computer-implemented method for predicting the binding of said peptide to any given HLA molecule. A preferred embodiment of this feature of the invention relates to the use of the software NetMHCpan and/or NetMHCII, or other alternatives as described herein, which are capable of determining (or predicting) the binding of any given, preferably nonameric, peptide in HLA or MHC molecules.

The proteasome cleavage prediction and HLA-binding prediction may be carried out in software designed to incorporate both modules in sequence, in order to provide an automatic determination of the number of PIRCHES.

In one embodiment the determination of PIRCHES as described herein relates to a method, wherein said PIRCHES are presented by shared HLA class I (PIRCHE I) and/or by shared HLA class II (PIRCHE II). In one embodiment the determination of PIRCHES as described herein relates to a method, wherein PIRCHE-I peptides have a predicted IC50 binding value of ≤10 µM, preferably ≤1000nM, more preferably ≤500nM. In one embodiment the determination of PIRCHES as described herein relates to a method, wherein PIRCHE-II peptides have a predicted IC50 binding value of ≤20 µM, preferably ≤5 µM, more preferably ≤1000nM. Other binding criteria may be applied for predicting whether a peptide will be presented by the HLA molecule. The provided values are based upon binding properties previously described in the literature, but different values may be derived or applied, if the PIRCHE can be reasonably be considered to bind and even customize it per presenting HLA.

For each donor-recipient pair, presentable recipient- or donor-specific HLA-derived peptides (PIRCHES) are identified. PIRCHE-I may preferably be identified in two steps:
1) In silico assessment of predicted processing of the amino acid sequences by the proteasome and transportation via the TAP channel is carried out, preferably using NetChop. NetChop is a commonly known software-based method for identifying the cleavage sites of the human proteasome based on sequence analysis. Nonameric peptides are preferably included in the analyses. In a preferred embodiment, the C-terminal cleavage potential is determined. The entire HLA protein is cleaved (preferably in silico) and all positions that are cleavable are marked. From the marked positions backwards, preferably nonameric peptides are identified that are analyzed for binding to class I. As alternatives to NetChop a variety of software-based approaches are known in the art that could be applied, such as MAPP, PaProc or those methods described in Lu et al (J Zhejiang Univ Sci B, 2013 Sep;14(9):816-28) or Ginodi et al (Bioinformatics, 2008 Feb 15;24(4):477-83).
2) Subsequently, peptides with a high probability of being processed (according to step 1) are tested for their capacity to be presented by HLA that are shared (matched) between the donor and recipient (preferably HLA-A, -B and -C) using NetMHCpan. NetMHCpan is a commonly known method for identifying and/or predicting the binding of peptides to any known MHC molecule using artificial neural networks. The method is trained on more than 150,000 quantitative binding data covering more than 150 different MHC molecules. Predictions can be made for HLA-A, B, C, E and G alleles. The prediction values can be given in nM IC50 values, or as %-Rank to a set of 200000 random natural peptides. Preferably peptides with IC50 binding values ≤500nM are chosen as relevant binders.

PIRCHE-II may be identified as follows:
Preferably nonameric binding cores of potential 15-meric HLA-DR, -DQ, and -DP binders may preferably be analysed with NetMHCIIPan 2.0, NetMHCII 1·0, or NetMHCII 2.2. NetMHCII is a commonly known method for predicting binding of peptides to HLA-DR, HLA-DQ, HLA-DP and mouse MHC class II alleles using artificial neuron networks. Predictions can be obtained for 14 HLA-DR alleles covering the 9 HLA-DR supertypes, six HLA-DQ, six HLA-DP, and two mouse H2 class II alleles. The prediction values are given in nM IC50 values, and as a %-Rank to a set of 1000000 random natural peptides. Peptides with IC50-binding values ≤1000nM are considered as relevant.

There are a number of alternative methods known in the art, which could be used for determination of HLA binding. SYFPEITHI (based on methods described in Rammensee et al. Immunogenetics 41, 178-228, 1995 and Rammensee et al, Landes Bioscience 1997) and BIMAS are well-known alternatives and are appropriate for class I binding but show some drawbacks in class II binding. Other alternatives relate to Tepitope (based on Sturniolo et al, 1999, Nat. Biotechnol. 17:555-561), TepitopePAN, EpicCapo, PAAQD, POPI, Propred and Multipred.

In one embodiment, per presenting shared-HLA allele, predicted binders derived from donor-HLA alleles are regarded as donor-self peptides, and recipient-HLA alleles regarded as recipient-self peptides, depending on the therapeutic setting, and thus excluded from the analyses. In general, for each donor-recipient pair, the number of presentable recipient- or donor-specific peptides (derived from the mismatched recipient-HLA allele and predicted to be presented by shared HLA) is counted in order to generate the number of PIRCHES.

In one embodiment the present invention relates to a method as described herein, wherein an analysis is carried out, for example the PIRCHES value is determined, for HLA subtypes HLA-A, HLA-B, HLA-C, HLA-DRB1, HLA-DQB1, HLA-DPB1, -DQA1, -DPA1, -G, -E, -F, MICA, MICB and/or KIR.

HLA typing is typically carried out on HLA-A, -B, -C, -DRB1 and -DQB1. These 5 alleles provide the basis for the commonly used terminology "9/10-matched", which refers to a 9/10-matched unrelated donor. In such a case 9 of the 10 alleles of these 5 genes show a match but one remaining allele does not match. The present invention therefore allows, in a preferred embodiment, determination of whether the use of material from such a 9/10-matched unrelated donor is safe, i. e. whether the mismatch is permissible for transplantation, in particular whether a 9/10 donor should be identified and selected for further typing.

The invention relates also to the method as described herein for finding permissible HLA-mismatches in donor samples where more mismatches are present than the common 9/10 scenario. One example of potential donors who are encompassed by the present invention are Haploidentical donors, who may be screened using the method described herein for permissible mismatched donor material. A haploidentical related donor, may be described as a donor who has a "50% match" to the patient. This type of donor can be a parent, sibling, or child. By definition, a parent of a patient will always be a haploidentical donor since half of the genetic material comes from each parent. There is a 50% chance that a sibling will be a haploidentical donor. Haploidentical HSCT offers many more people the option of HCT as 90% of patients have a haploidentical family member. Other advantages include: immediate donor availability; equivalent access for all patients regardless of ethnic background; ability to select between multiple donors; and ability to obtain additional cells if needed. Alternatively, cord blood units (CBUs) may not show a high level of HLA-matching, but still be suitable for transplantation if the mismatches are permissible as determined by the method as described herein.

Considering that the method as described herein relies to some extent on shared HLA, the minimum HLA-match is one allelic match. Although this is unlikely to occur for HSCT, such a scenario will frequently arise for organ transplantation, due to the limited number of donor material available. The invention may therefore be carried out on mismatched samples with a minimum of one allelic match. The mismatched donor may therefore relate to a 1/10, 2/10, 3/10, 4/10, 5/10, 6/10, 7/10, 8/10, 9/10 or 10/10 (DP mismatch) match. If additional alleles are tested, the donor may also show any other kind of mismatch, whereby at least one allelic match is present. Preferred for the transplantation are donors with significant HLA-matches. If additional alleles are subjected to typing the donor could therefore be for example 11/12-matched.

In one embodiment the present invention relates to a method as described herein for predicting the best donor out of multiple HLA-mismatched donors. In one embodiment the present invention relates to a method as described herein comprising additionally the analysis and comparison of multiple HLA-mismatched donors for the purpose of predicting alloreactivity between multiple donors, for example between two cord blood units and/or between a matched and an HLA-mismatched donor. As transplantation, especially of stem cells or cord blood, becomes a more reliable and preferred therapeutic option, at times multiple donor samples must be used in order to provide sufficient material for the transplantation. In light of this, the present method enables a "multi-donor" analysis, thereby comparing not only donor-recipient matches, but additionally or alternatively assessing donor-donor matches, in order to determine in advance of additional donor sample may be compatible with the recipient in addition to each other. The present method therefore enables fast and reliable "three-way" compatibility assessment without the complications of the laboratory-based methods of the past.

The method of the invention enables a number of beneficial technical and secondary effects. Through the method as presently described allogeneic donor material may be selected that is associated with a relatively low risk of an unwanted immune reaction. In light of this effect, the invention saves time due to the automated and/or computer-implementation, reduces health risk, reduces medical costs for high resolution sequencing and potentially failed transplantations, increases the pool of possible donor material for any given recipient by enabling the analysis of low resolution or intermediate resolution typed donors and enables previously untreatable patients (those for example have very rare HLA-alleles) to be treated with allogeneic material due to the risk reduction by selecting permissible mismatches.

### DETAILED DESCRIPTION OF THE INVENTION

"HLA typing" means the identification of an HLA allele of a given locus (HLA-A, HLA-B, HLA-C, HLA-DRB1, HLA-DQB1, etc...). Samples may be obtained from blood or other body samples from donor and/or recipient, which may subsequently be analysed.

Serotyping (identification of the HLA protein on the surface of cells using antibodies) is the original method of HLA typing and is still used by some centres. Phenotyping relates to the serological approach for typing. This method is limited in its ability to define HLA polymorphism and is associated with a risk of misidentifying the HLA type. Modern molecular methods are more commonly used to genotype an individual. With this strategy, PCR primers specific to a variant region of DNA are used (called PCR SSP). If a product of the right size is found, the assumption is that the HLA allele/ allele group has been identified. PCR-SSO may also be used incorporating probe hybridisation. Reviews of technical approaches towards HLA typing are provided in Erlich H, Tissue Antigens, 2012 Jul;80(1):1-11 and Dunn P, Int J Immunogenet, 2011 Dec;38(6):463-73. Gene sequencing may be applied, and relates to traditional methods, such as Maxam-Gilbert sequencing, Chain-termination methods, advanced methods and de novo sequencing such as shotgun sequencing or bridge PCR, or the so-called "next-generation" methods, such as massively parallel signature sequencing (MPSS), 454 pyrosequencing, Illumina (Solexa) sequencing, SOLiD sequencing, or other similar methods.

With respect to HLA typing, samples obtained from the donor themselves and/or from donor material before, during or after isolation/preparation for transplantation, may be used for HLA-typing and subsequent comparison to the HLA-typing data from the recipient. For example, HLA-typing of the donor themselves, for example by analysing a saliva, blood or other bodily fluid or material sample for HLA information, may occur, and optionally additionally or alternatively, the material obtained from the donor intended for transplantation (donor material) may be analysed for the same and/or complementary HLA type characteristics during HLA-typing.

An "HLA-mismatch" is defined as the identification of different alleles in donor and recipient, which are present at any given loci.

According to the present invention, the term "two-field specific HLA protein typing" relates to the standardised HLA naming system developed in 2010 by the WHO Committee for Factors of the HLA System. According to the HLA naming system, the nomenclature is as follows: HLA-Gene*Field1:Field2:Field3:Field4-Suffix. Gene relates to the HLA locus (A, B, C, DRB1, DQB1, etc...). Field 1 relates to the allele group. Field 2 relates to the specific HLA protein. Field 3 relates to a synonymous DNA substitution within the coding region. Field 4 is used to show differences in a non-coding regions. The suffix is used to denote particular characteristics in gene expression.

The "two-field" within "two-field specific HLA protein typing" relates to the presence of HLA typing information for Fields 1 and 2 according to the standardized WHO nomenclature used herein. This typing is commonly referred to as "high resolution typing" or "specific HLA protein typing", as the typing provides information on the specific protein sequence relevant for the immune responses dealt with herein.

The term "HLA allele group" typing relates to typing information that is provided for only the HLA gene (locus) and Field1. This typing is commonly referred to as "low resolution" typing. According to the present invention any serotype would be converted to a molecular "low resolution" typing.

The term "intermediate resolution" typing relates to some kinds of typing information where the presence of some alleles, or allele strings, have been defined in the patient/donor by the typing method used. The NMDP nomenclature is one method of describing this level of typing. For example A*01:AB relates to alleles 01:01 or 01:02 (01:01/01:02) and the patient/donor is one of these two alleles. The NMDP allele codes are in some cases generic, wherein one code may encompass a number of possible HLA sequences (alleles) at any given HLA locus. Although the donor material may be HLA typed, a number of NMDP codes do not provide information on which specific protein is in fact present at any given HLA locus. This form of typing information is referred to as intermediate resolution typing.

According to the present invention, the "two-field specific HLA protein typing" may relate to an "unambiguous two-field specific HLA protein typing". The term "unambiguous" within "unambiguous two-field specific HLA protein typing" is commonly understood as described in Adams et al (Journal of Translational Medicine 2004, 2:30). An "unambiguous" typing therefore contains no ambiguities as commonly understood by a skilled person. Unambiguous typing information is more extensive than regular two field typing as it removes additional uncertainties regarding the particular HLA type of the donor/recipient. In one embodiment of the invention the method is carried out using candidate donors, for whom no unambiguous two-field specific HLA protein typing information is available.

There exist three main types of ambiguous typing results obtained with sequence based typing. The first type of ambiguity is when a heterozygous sequence can be explained by more than one possible pair of alleles within the region analyzed. The second exists when alleles are defined by a polymorphism outside the region analyzed. In addition to these two situations, a third type of ambiguity arises when an allele has an incomplete sequence in the region analyzed. The prevalence of ambiguities in HLA typing relates to the nature of polymorphisms which exists in the sequence of the major histocompatibility complex (MHC) Class I and Class II genes. The majority of polymorphisms that distinguish one MHC allele from another are often due to gene conversion, recombination and exon shuffling events. Due to this, polymorphic motifs at given positions are generally shared among several alleles. Sequence-based typing involves PCR amplification and sequencing of specific HLA exons, which are known to be polymorphic, from genomic DNA. For each HLA locus both alleles are amplified and sequenced; therefore, it is not always possible to determine exactly which two alleles were responsible for sequence results. The second type of ambiguity relates to defining a polymorphism outside the region analyzed. For example, many HLA-A alleles are defined by a polymorphism located in Exon 4. Traditionally, for Class I typing most laboratories only sequence Exon 2 and Exon 3; for Class II typing most laboratories only sequence Exon 2. This approach has been the standard due to the functional relevance of this region which defines the peptide groove of Class I and Class II molecules, respectively. However, some Class I alleles have identical sequences across Exons 2 and 3. To resolve these alleles it is necessary to analyze the gene at the region where they differ. Finally, an ambiguity may be due to incomplete sequence information, because not all alleles have been sequenced for the same exons. For some alleles the entire sequence is not known in the region that is amplified.

The P and G nomenclature system was developed to report ambiguities. Alleles having nucleotide sequences that encode the same protein sequence for the peptide binding domains (exon 2 and 3 for HLA class I and exon 2 only for HLA class II alleles) are designated by an upper case 'P' which follows the 2 field allele designation of the lowest numbered allele in the group e.g. A*01:01P. Alleles that have identical nucleotide sequences across the exons encoding the peptide binding domains (exon 2 and 3 for HLA class I and exon 2 only for HLA class II alleles), are designated by an upper case 'G' which follows the first 3 fields of the allele designation of the lowest numbered allele in the group e.g. A*01:01:01G.

The NMDP and P/G nomenclature can be used in the invention described herein.

"Prediction" means a statement about possible events in the future. The term "forecast" may also be used. The "prediction" in the sense of the present invention represents an assessment of the likelihood or risk of an immune response occurring after transplantation. On the basis of the prediction, or risk assessment, valuable information is obtained in advance of a potentially harmful event, which can be used to determine further therapeutic options.

The term "Immune response" in the context of the present invention relates to an immune response as commonly understood by one skilled in the art. An immune response may be understood as a response from a part of the immune system to an antigen that occurs when the antigen is identified as foreign, which preferably subsequently induces the production of antibodies and/or lymphocytes capable of destroying or immobilising the "foreign" antigen or making it harmless. The immune response of the present invention may relate either to a response of the immune system of the recipient against the transplanted material, or an immune response effected by cells of the transplanted cells, tissues, or organs, whereby for example in GVHD T-cells of the transplanted material react against and/or attack recipient antigens or tissue. The immune response may be a defence function of the recipient that protects the body against foreign matter, such as foreign tissue, or a reaction of immune cells of the transplanted material against recipient cells or tissue.

The human leukocyte antigen (HLA) system is the major histocompatibility complex (MHC) in humans. The super locus contains a large number of genes related to immune system function in humans. This group of genes resides on the short arm of chromosome 6, and encodes cell-surface antigen-presenting proteins and has many other functions. The proteins encoded by certain genes are also known as antigens, as a result of their historic discovery as factors in organ transplants. The major HLA antigens are essential elements for immune function. HLAs corresponding to MHC class I (A, B, and C) present peptides from inside the cell (including viral peptides if present). These peptides are produced from digested proteins that are broken down in the proteasomes. In general, these particular peptides are small polymers, about 9 amino acids in length. Foreign antigens attract killer T-cells (also called CD8 positive- or cytotoxic T-cells) that destroy cells. HLAs corresponding to MHC class II (DP, DM, DOA, DOB, DQ, and DR) present antigens from outside of the cell to T-lymphocytes. These particular antigens stimulate the multiplication of T-helper cells, which in turn stimulate antibody-producing B-cells to produce antibodies to that specific antigen.

"HLA" refers to the human leukocyte antigen locus on chromosome 6p21, consisting of HLA genes (HLA-A, HLA-B, HLA-C, HLA-DRB1, HLA-DQB1, etc... ) that are used to determine the degree of matching, for example, between a recipient and a donor of a tissue graft. "HLA allele" means a nucleotide sequence within a locus on one of the two parental chromosomes. An allele is a variant of the nucleotide (DNA) sequence at a locus, such that each allele differs from all other alleles by at least one (single nucleotide polymorphism, SNP) position. Most of these changes result in a change in the amino acid sequences that result in slight to major functional differences in the protein.

The donor is commonly understood to be an individual or multiple individuals (for example in the case of where multiple samples or preparations, such as cord blood units (CBUs) are required for an effective therapeutically relevant amount of donor material for the transplantation) who provide donor material, or in other words biological material, such as but not limited to cell(s), tissues, organs, or other bodily parts, fluids and/or preparations, for transplantation or implantation in a recipient. In the context of the present invention the term transplantation may encompass implantation as understood by a skilled person. References to the donor, or HLA-typing of the donor, may also refer to donor material, or HLA-typing of the donor material, respectively.

The subject recipient of the method is typically a mammal, preferably a human. The recipient is typically a patient suffering from a disorder characterised by the need for transplantation, such as organ failure necessitating a transplant. By the term "organ", it is meant to include any bodily structure or group of cells which may contain different tissues that are organized to carry out a specific function of the body, for example, the heart, lungs, brain, kidney, skin, liver, bone marrow, etc. In one embodiment the graft is an allograft, i.e. the individual and the donor are of the same species. The subject may also suffer from a condition that could be treated by the transplantation of cells, even when the disorder itself is not defined by a lack or loss of function of a particular subset of endogenous cells. Some disorders may be treatable by the transplantation of certain kinds of stem cells, whereby the native or endogenous pool of such cells are not necessarily non-functional in the recipient.

The method of the invention is particularly applicable to patients who are about to receive or are predicted to require a cell, tissue or organ transplant, to predict the likelihood of unwanted immune response, such as graft damage or rejection, and/or immune origin damage to non-graft tissue. For example, the patient may be expected to receive a transplant in the next one, two, three, four, five, six, or twelve months. Alternatively, the assay is particularly applicable to individuals who have received a transplant to predict the likelihood of immune origin graft damage or rejection, and/or immune origin damage to non-graft tissue. Post-transplant, the method is particularly applicable to patients who show evidence of chronic organ dysfunction (of the graft organ) or possible graft versus host disease (GVHD), particularly chronic GVHD and particularly in cases wherein the graft is a bone marrow transplant.

Transplantation is the moving of cells, tissue or an organ from one body (donor) to another (recipient or patient), or from a donor site to another location on the patient's own body, or from stored donor material to a recipients body, for the purpose of replacing the recipient's damaged or absent organ, or for the purpose of providing stem cells, other cells, tissues or organs capable of providing a therapeutic effect.

Allogeneic transplantation or Allotransplantation is the transplantation of cells, tissues, or organs, to a recipient from a genetically non-identical donor of the same species. The transplant is called an allograft, allogeneic transplant, or homograft. Most human tissue and organ transplants are allografts. Allografts can either be from a living or cadaveric source. Generally, organs that can be transplanted are the heart, kidneys, liver, lungs, pancreas, intestine, and thymus. Tissues include bones, tendons (both referred to as musculoskeletal grafts), cornea, skin, heart valves, nerves and veins. Worldwide, the kidneys are the most commonly transplanted organs, followed by the liver and then the heart. Cornea and musculoskeletal grafts are the most commonly transplanted tissues.

The invention also encompasses use of the method in the context of screening organs, cells, or tissues produced via regenerative medicine, for example reconstructed donor material that has been constructed ex vivo and is intended for transplantation. Stem cell technologies enable the production of a number of medically relevant cell types or tissues ex vivo. The present invention could therefore also be applied in screening allogeneic material that has been produced by biotechnological and/or tissue engineering methods for its suitability in transplantation.

Hematopoietic stem cell transplantation (HSCT) is the transplantation of multipotent hematopoietic stem cells, usually derived from bone marrow, peripheral blood, or umbilical cord blood. It is a medical procedure common in the fields of hematology and oncology, most often performed for patients with certain cancers of the blood or bone marrow, such as multiple myeloma or leukemia. In these cases, the recipient's immune system is usually destroyed with radiation or chemotherapy before the transplantation. Infection and graft-versus-host disease are major complications of allogenic (also referred to as allogeneic) HSCT.

Stem cells are to be understood as undifferentiated biological cells, that can differentiate into specialized cells and can divide (through mitosis) to produce more stem cells. Highly plastic adult stem cells are routinely used in medical therapies, for example in bone marrow transplantation. Stem cells can now be artificially grown and transformed (differentiated) into specialized cell types with characteristics consistent with cells of various tissues such as muscles or nerves through cell culture. Embryonic cell lines and autologous embryonic stem cells generated through therapeutic cloning have also been proposed as promising candidates for future therapies. The potential stem cell transplantation may relate to any given stem cell therapy, whereby a number of stem cell therapies exist. Medical researchers anticipate that adult and embryonic stem cells will soon be able to treat cancer, Type 1 diabetes mellitus, Parkinson's disease, Huntington's disease, Celiac disease, cardiac failure, muscle damage and neurological disorders, and many others.

Also known as somatic stem cells and germline stem cells, stem cells can be found in children, as well as adults. Pluripotent adult stem cells are rare and generally small in number but can be found in a number of tissues including umbilical cord blood. Bone marrow has been found to be one of the rich sources of adult stem cells which have been used in treating several conditions including Spinal cord injury, Liver Cirrhosis, Chronic Limb Ischemia and Endstage heart failure. Adult stem cells may be lineage-restricted (multipotent) and are generally referred to by their tissue origin (mesenchymal stem cell, adipose-derived stem cell, endothelial stem cell, dental pulp stem cell, etc.).

Multipotent stem cells are also found in amniotic fluid. These stem cells are very active, expand extensively without feeders and are not tumorigenic. Amniotic stem cells are multipotent and can differentiate in cells of adipogenic, osteogenic, myogenic, endothelial, hepatic and also neuronal lines. It is possible to collect amniotic stem cells for donors or for autologous use.

Cord blood-derived multipotent stem cells display embryonic and hematopoietic characteristics. Phenotypic characterization demonstrates that (CB-SCs) display embryonic cell markers (e.g., transcription factors OCT-4 and Nanog, stage-specific embryonic antigen (SSEA)-3, and SSEA-4) and leukocyte common antigen CD45, but that they can be negative for blood cell lineage markers. Additionally, CB-SCs display very low immunogenicity as indicated by expression of a very low level of major histocompatibility complex (MHC) antigens and failure to stimulate the proliferation of allogeneic lymphocytes.

HSC are typically available from bone marrow, Peripheral blood stem cells, Amniotic fluid, or Umbilical cord blood. In the case of a bone marrow transplant, the HSC are removed from a large bone of the donor, typically the pelvis, through a large needle that reaches the centre of the bone. The technique is referred to as a bone marrow harvest and is performed under general anaesthesia. Peripheral blood stem cells are a common source of stem cells for allogeneic HSCT. They can be collected from the blood through a process known as apheresis. The donor's blood is withdrawn through a sterile needle in one arm and passed through a machine that removes white blood cells. The red blood cells may be returned to the donor. The peripheral stem cell yield may be boosted with daily subcutaneous injections of Granulocyte-colony stimulating factor, serving to mobilize stem cells from the donor's bone marrow into the peripheral circulation.

It is also possible to extract hematopoietic stem cells from amniotic fluid. Umbilical cord blood is obtained from an infant's Umbilical Cord and Placenta after birth. Cord blood has a higher concentration of HSC than is normally found in adult blood. However, the small quantity of blood obtained from an Umbilical Cord (typically about 50 mL) makes it more suitable for transplantation into small children than into adults. Multiple units could however be used. Newer techniques using ex-vivo expansion of cord blood units or the use of two cord blood units from different donors allow cord blood transplants to be used in adults. Cord blood can be harvested from the umbilical cord of a child being born.

Unlike other organs, bone marrow cells can be frozen (cryopreserved) for prolonged periods without damaging too many cells. This is a necessity with autologous HSC because the cells are generally harvested from the recipient months in advance of the transplant treatment. In the case of allogeneic transplants, fresh HSC are preferred in order to avoid cell loss that might occur during the freezing and thawing process. Allogeneic cord blood is typically stored frozen at a cord blood bank because it is only obtainable at the time of childbirth. To cryopreserve HSC, a preservative, DMSO, must be added, and the cells must be cooled very slowly in a controlled-rate freezer to prevent osmotic cellular injury during ice crystal formation. HSC may be stored for years in a cryofreezer, which typically uses liquid nitrogen. In light of this, the invention may relate to typing and risk assessment of donor material already stored as described herein, before being considered for transplantation.

The invention encompasses the assessment of risk of an immune reaction, preferably a pre-transplantation risk assessment, whereby any given organ or tissue may be considered as donor material intended for transplantation. For example, kidney transplantation or renal transplantation is the organ transplant of a kidney into a patient, for example with end-stage renal disease. Kidney transplantation is typically classified as deceased-donor (formerly known as cadaveric) or living-donor transplantation depending on the source of the donor organ. Living-donor renal transplants are further characterized as genetically related (living-related) or non-related (living-unrelated) transplants, depending on whether a biological relationship exists between the donor and recipient.

Alloreactivity is defined as the reaction of a lymphocyte or antibody with an alloantigen, which may be understood as an antigen from foreign material. Alloantigen recognition may occur via direct or indirect alloantigen recognition, by which T cells may recognize alloantigens and potentially lead to transplant rejection after an organ transplant.

Graft-versus-host disease (GVHD) is a relatively common complication following an allogeneic cell, tissue or organ transplant. It is commonly associated with stem cell or bone marrow transplant but the term also applies to other forms of tissue graft or organ transplant. Immune cells (typically white blood cells) in the tissue (the graft) recognize the recipient (the host) as "foreign". The transplanted immune cells then attack the host's body cells. GVHD can also occur after a blood transfusion if the blood products used have not been irradiated.

Host-versus-Graft disease (GVHD) is a relatively common complication following an allogeneic cell, tissue or organ transplant. It is commonly associated with solid organ transplants but the term also applies to other forms of tissue graft or organ transplant. Immune cells (typically white blood cells) of the recipient (host) recognize the donor (the graft) as "foreign". The recipient immune response (cells and antibodies) then attack the graft cells.

### TABLES

The invention is described by the following Tables. The Tables provided herein represent support for particular embodiments of the invention and are not intended to limit the scope of the invention.

**Table 1:** The median number of Pirche I and II score groups for comparisons between Patients 1 and 2 with 3 donor types with a 1A mismatch together with the combined number of Pirches are shown.

**Table 2:** The median number of Pirche I and II score groups for comparisons between Patients 1 to 4 with donors for multiple sequences of multiple allele groups are shown. The combined number of Pirche are shown, in addition to the percentage of alleles per allele group with either less than 11 or less than 6 as a median Pirche score (value).

**Table** 3: A summary of the alleles groups having the lowest median Pirche scores for patients 1, 2, 3 and 4 are shown. The combined number of Pirche are shown, in addition to the percentage of alleles per allele group with either less than 11 or less than 6 as a median Pirche score.

### EXAMPLES

The invention is described by the following examples. The examples provided herein represent practical support for particular embodiments of the invention. These are not intended to limit the scope of the invention. The examples are to be considered as providing a description of possible and potentially preferred embodiments that demonstrate the relevant technical working of one or more non-limiting embodiments.

The invention enables the following embodiments, which are supported by the examples below:
- When a fully matched donor is not available, the present invention enables use of the PIRCHE platform for transplant centres (TC) and unrelated stem cell registries/ cord bank registries to help them identify the most suitable mismatched donor:
- When suitable mismatched donors have been identified, the method enables the user to determine which donor is the best to use when any other clinical considerations e.g. blood group, CMV status, age, gender have been taken into account. The method also enables the user to determine which mismatched donors on the search are the best to consider for further testing.
- Enables the user to identify the best locus to mismatch so the search analysis can focus on potential donors with the desired mismatches.

The previous PIRCHE platform requires the patient and donor to be high resolution typed. This is not necessarily the policy of the TC, so either the TC will not be inclined to use the platform or they will incur extra expense having to perform high resolution typing.

### Test 1:

The present example therefore outlines an approach to calculate a PIRCHE estimate which is the median PIRCHE score for the patient/ donor comparison.

For a given patient typing:
Patient 1:
A*02:01,A*31:01,B*51:01,B*15:01,C*03:04,C*15:02,DRB1*14:54,DQB1*05:03 3 donor types which were a 1A mismatch at the low resolution (LR) were identified.
A*02:01,**A*01**,B*51:01,B*15:01,C*03:04,C*15:02,DRB1*14:54,DQB1*05:03
A*02:01,**A*03**,B*51:01,B*15:01,C*03:04,C*15:02,DRB1*14:54,DQB1*05:03
A*02:01,**A*68**,B*51:01,B*15:01,C*03:04,C*15:02,DRB1*14:54,DQB1*05:03

For each of the donor types the PIRCHE determination was carried out matching for a large number of the possible known sequences at the mismatched locus to determine the individual allele PIRCHE scores. The inventor then determined how many different PIRCHE score groups there were for each LR mismatch (considered the combined PIRCHE score). All the LR mismatch donors gave 3 PIRCHE score groups and the number of alleles in each group is shown in Table 1. For A*03 allele mismatch donor comparison the three PIRCHE score groups consisted of 19, 21 and 23 peptide differences. There were 138 A*03 alleles which had 19 peptide differences with the patient, 5 A*03 alleles with 21 peptide differences and 45 A*03 alleles with 23 peptide differences.

The above comparison was repeated but with a slightly different patient typing:
Patient 2:
A*02:01,A*01:01,B*51:01,B*15:01,C*03:04,C*15:02,DRB1*14:54,DQB1*05:03

The results are shown in Table 1.

The median number of PIRCHE I and II score groups for each comparison together with the combined number are shown in Table 1 for each patient. Using the median scores to compare the allele groups, there does not appear to be any difference between A*01, A*03 or A*68 as a mismatch for patient 1, the median scores being 19, 19 and 23 respectively. Interestingly for patient 2 there is very little difference between A*01 (the patient's own allele group) and A*03 as an allele group mismatch, whereas A*68 allele group does give a higher median PIRCHE score (median scores being 1, 3 and 13 respectively). Based on this comparison a conclusion that could be drawn is that for patient 2, an A*03 allele group mismatched donor would be a more suited donor than an A*68 allele group mismatched donor, whereas for patient 1 A*01, A*03 or A*68 are not suitable allele group mismatches.

This approach can be used for patients to give an indication which donors with intermediate or low resolution typing on a search would give a probability of the lowest PIRCHE score.

The above approach can also be used for patients to determine which loci and the allele group would give a probability of the lowest PIRCHE score.

The above can be incorporated into a computer algorithm for automatic execution.

### Test 2:

The inventor performed additional simulations with A*11, A*30 and A*32 allele group mismatched donors but this did not demonstrate a significant difference between the allele groups. Since the allele groups chosen for the simulations were picked at random it was decided to test patients 1 and 2 against all the A allele groups. The results demonstrate that both patients have clearly different allele groups with low overall median PIRCHE scores.

Patient 1: A*02:01,A*31:01,B*51:01,B*15:01,C*03:04,C*15:02,DRB1*14:54,DQB1*05:03

Donors with an A mismatch of the A*29, A*33 allele groups have an overall median PIRCHE score of 2.

Patient 2: A*02:01,A*01:01,B*51:01,B*15:01,C*03:04,C*15:02,DRB1*14:54,DQB1*05:03

Donors with an A mismatch of the A*03, A*11, A*30 or A*36 allele groups have an overall median PIRCHE score of 3, 4, 5, and 1 respectively. The allele groups A*23 has a marginally higher value of 9.

Considering the allele groups in these patient cases, the number of alleles (or specific sequences) under each allele group are not necessarily normally distributed with respect to the number of PIRCHE scores. For Patient 1, the PIRCHE scores of each of the sequences in the A33 allele group are distributed across six PIRCHE score groups, whereas for the A29 allele group there are three PIRCHE scores yet the median PIRCHE I and II is 2 for both allele groups.

The chances of having either high or low PIRCHE scores for any given specific allele appear to be similar for both these allele groups.

However, when one examines Patient 2, A36 appears to be the best allele. There is a 100% chance to have PIRCHE scores of 1 or 2 from any of the protein sequence variants within the allele group.

This example shows clearly that one could qualify allele groups with respect to the distribution of PIRCHE scores for each of the protein sequence variants within the allele group.

Alternatively or in addition, a cut off regarding the percentage of alleles per allele group with less than a specified median PIRCHE score across all protein sequence variants of the allele group can be applied (see Table 2; median PIRCHE score <11 and <6 used for demonstration purposes). This estimates the probability of a donor with a particular allele group mismatch having a low PIRCHE score when typed at high resolution.

Additional simulations were run for a further two patients (patients 3 and 4) against all the A allele groups (see Table 2). For patient 3 the A*24 allele group and patient 4 the allele groups A*01 and A*36 gave the lowest median PIRCHE score. A summary of the allele groups with the lowest median PIRCHE scores for patients 1 to 4 are shown in Table 3.

Such classification of allele groups provide clear indications for selecting a candidate donor for high resolution typing.

### Example regarding analysis with a recipient typed at low resolution:

All current PIRCHE solutions rely on the patient being typed to a high resolution. For HSCT and particularly solid organ transplantation, it would useful to be able to apply PIRCHE for patients (recipients) with a low or intermediate typing.

For HSCT patients the current GVHD direction is be used to calculate PIRCHE scores but for solid organ transplants the HVG direction would have to be used.

### HSCT patients with a low/intermediate resolution typing:

For HSCT only one locus is usually considered for mismatching but the principle outlines below can be used for multiple mismatched loci.

Virtual patients would be created electronically representing all or a defined number of allele combinations of the patients low/intermediate resolution type. This would give Px virtual patients (x = 1 to n).

For each virtual patient (recipient), virtual donors differing by just one allele at the mismatch locus would be created. The class I, class II and I/II PIRCHE scores for each virtual donor would be calculated and the median PIRCHE score calculated for each allele group.

For each allele group the mean class I, class II and I/II PIRCHE scores of all the virtual patients (Px) would be calculated and used for comparison with other allele group results.

This approach would give the TC and unrelated stem cell registries/ cord bank registries an estimation of which allele group would be the best to mismatch to give a probable low PIRCHE value based on a patients low/ intermediate typing.

This approach can be used to select the most appropriate haplo-identical donor for further high resolution typing or for transplant.

For intermediate typing the virtual donors created above would only have to represent the alleles in the patients (recipients) allele typing string.

### Solid Organ transplant patients with a low/intermediate resolution typing:

The above principles would be used but the PIRCHE values would have to be calculated for the recipient binding donor peptide (HvG) and all the recipient HLA molecules are considered as peptide presenting alleles - shared and non-shared alleles.

The majority of recipients and donors in solid organ transplantation are HLA typed to the low or intermediate level. Low resolution may be a HLA allele group typing (e.g. A*02) or be the serological equivalent (e.g. B62).

Virtual patients and donors would be created with all known allele combinations of the low/ intermediate typing or with a predefined number of alleles. The predefined alleles may be by way of example, but not limited to, one of the following:
- Defined by user criteria as in a "look up" table. For example serological B62 could be defined as alleles B*15:01 and B*15:28.
- Defined at the most likely allele.
- Defined by an allele frequency table.
- Defined as the alleles which constitute a defined percentage of the alleles of that allele group in that population. For example A*02:01 and A*02:05 may represent the A*02 alleles observed in 95% of the A*02s in the population and used as the predefined alleles. Donor population is defined, but not limited to, a large data set of donor HLA types. Recipient population is defined, but not limited to, frequencies seen in available frequency tables for the recipient ethnic group.
- Defined as a selection the leading alleles in the allele string of an intermediate typing. For example for the allele string
   B*35:01/35:07/35:12/35:19/35:20/35:24/35:27/35:29/35:30/35:32/35:40N/35:41/35:48/35: 50/35:52/35:53N/35:57/35:64/35:68/35:77 alleles B*35:01/35:07/35:12/35:19 may be used as predefined alleles.

PIRCHE calculations are performed, but not limited to, those already described.

This approach can be used to select acceptable mismatches for transplants using deceased donors and to select the most appropriate live donor when more than one candidate donor is available.

This approach can be used to determine the relative risk of recipient sensitization to mismatches to aid transplant centres to define unacceptable or acceptable mismatches. Unacceptable mismatches are defined, but not limited to, mismatches that will not be considered for the patient. Acceptable mismatches are defined, but not limited to, mismatches that will be considered for the patient but are considered a higher risk.

**Table 1.**

| **Patient 1** | **A*02:01** | | **B*51:01** | **C*03:04** | **DRB1*14:54** | **DQB1*05:03** | | | | | | **Median PIRCHE score** | | |
|---|---|---|---|---|---|---|---|---|---|---|---|---|---|---|
| | **A*31:01** | | **B*15:01** | **C*15:02** | **-** | **-** | | | | | | | | |
| | | Total **#** Pirche score groups | **#** Pirche peptides | **#** Pirche peptides | **#** Pirche peptides | | | | | | | I | II | I & II |
| A*01 alleles | Pirche scores (combined) | 3 | 19 | 21 | 23 | | | | | | | 13 | 6 | 19 |
| | **#** alleles/ pirche score | | 96 | 8 | 53 | | | | | | | | | |
| A*03 alleles | Pirche scores (combined) | 3 | 19 | 21 | 23 | | | | | | | 13 | 6 | 19 |
| | **#** alleles/ pirche score | | 138 | 5 | 45 | | | | | | | | | |
| A*68 alleles | Pirche scores (combined) | 3 | 17 | 20 | 23 | | | | | | | 17 | 6 | 23 |
| | **#** alleles/ pirche score | | 1 | 1 | 110 | | | | | | | | | |
| | | | | | | | | | | | | | | |

| **Patient 2** | **A*02:01** | | **B*51:01** | **C*03:04** | **DRB1*14:54** | **DQB1*05:03** | | | | | | **Median PIRCHE score** | | |
|---|---|---|---|---|---|---|---|---|---|---|---|---|---|---|
| | **A*01:01** | | **B*15:01** | **C*15:02** | **-** | **-** | | | | | | | | |
| | | Total **#** Pirche score groups | **#** Pirche peptides | **#** Pirche peptides | **#** Pirche peptides | **#** Pirche peptides | **#** Pirche peptides | **#** Pirche peptides | **#** Pirche peptides | **#** Pirche peptides | **#** Pirche peptides | I | II | I & II |
| A*01 alleles | Pirche scores (combined) | 7 | 0 | 1 | 2 | 6 | 7 | 10 | 14 | | | 1 | 0 | 1 |
| | **#** alleles/ pirche score | | 62 | 23 | 12 | 1 | 3 | 1 | 53 | | | | | |
| A*03 alleles | Pirche scores (combined) | 9 | 2 | 3 | 4 | 5 | 7 | 9 | 10 | 11 | 14 | 3 | 0 | 3 |
| | **#** alleles/ pirche score | | 4 | 120 | 3 | 10 | 1 | 2 | 1 | 2 | 45 | | | |
| A*68 alleles | Pirche scores (combined) | 3 | 12 | 13 | 14 | | | | | | | 10 | 3 | 13 |
| | **#** alleles/ pirche score | | 53 | 4 | 55 | | | | | | | | | |

**Table 2.**

| **Patient 1** | **A*02:01** | | **B*51:01** | **C*03:04** | **DRB1*14:54** | **DQB1*05:03** | | | | | | | | **Median PIRCHE score** | | | | | |
|---|---|---|---|---|---|---|---|---|---|---|---|---|---|---|---|---|---|---|---|
| | **A*31:01** | | **B*15:01** | **C*15:02** | **-** | **-** | | | | | | | | | | | | | |
| | | Total # Pirche score groups | # Pirche peptides | # Pirche peptides | # Pirche peptides | # Pirche peptides | # Pirche peptides | # Pirche peptides | # Pirche peptides | # Pirche peptides | # Pirche peptides | # Pirche peptides | # Pirche peptides | I | II | I & II | | % alleles <11 | % alleles <6 |
| A1 alleles | Pirche scores (combined) | 3 | 19 | 21 | 23 | | | | | | | | | 13 | 6 | 19 | 01:01 = 19 | 0% | 0% |
| | # alleles/ pirche score | | 95 | 7 | 53 | | | | | | | | | | | | | | |
| A2 alleles | Pirche scores (combined) | 4 | 16 | 17 | 20 | 23 | | | | | | | | 17 | 6 | 23 | 02:01 = 23 | 0% | 0% |
| | # alleles/ pirche score | | 1 | 1 | 1 | 503 | | | | | | | | | | | | | |
| A3 alleles | Pirche scores (combined) | 3 | 19 | 21 | 23 | | | | | | | | | 13 | 6 | 19 | 03:01 = 19 | 0% | 0% |
| | # alleles/ pirche score | | 138 | 5 | 45 | | | | | | | | | | | | | | |
| A11 alleles | Pirche scores (combined) | 4 | 13 | 19 | 21 | 23 | | | | | | | | 13 | 6 | 19 | 11:01 = 19 | 0% | 0% |
| | # alleles/ pirche score | | 1 | 108 | 11 | 72 | | | | | | | | | | | | | |
| A23 alleles | Pirche scores (combined) | 2 | 21 | 23 | | | | | | | | | | 15 | 6 | 21 | 23:01 = 21 | 0% | 0% |
| | # alleles/ pirche score | | 45 | 19 | | | | | | | | | | | | | | | |
| A24 alleles | Pirche scores (combined) | 4 | 15 | 19 | 21 | 23 | | | | | | | | 15 | 6 | 21 | 24:01 = 23 | 0% | 0% |
| | # alleles/ pirche score | | 1 | 1 | 174 | 105 | | | | | | | | | | | | | |
| A25 alleles | Pirche scores (combined) | 2 | 20 | 23 | | | | | | | | | | 14 | 6 | 20 | 25:01 = 20 | 0% | 0% |
| | # alleles/ pirche score | | 15 | 10 | | | | | | | | | | | | | | | |
| A26 alleles | Pirche scores (combined) | 2 | 20 | 23 | | | | | | | | | | 14 | 6 | 20 | 26:01 =20 | 0% | 0% |
| | # alleles/ pirche score | | 68 | 34 | | | | | | | | | | | | | | | |
| **A29** alleles | **Pirche** scores **(combined)** | 3 | 2 | 12 | 23 | | | | | | | | | 2 | 0 | 2 | 29:01 **= 2** | 52% | 52% |
| | # **alleles/ pirche** score | | 34 | 1 | 31 | | | | | | | | | | | | | | |
| A30 alleles | Pirche scores (combined) | 3 | 19 | 21 | 23 | | | | | | | | | 13 | 6 | 19 | 30:01 = 19 | 0% | 0% |
| | # alleles/ pirche score | | 55 | 6 | 24 | | | | | | | | | | | | | | |
| A31 alleles | Pirche scores (combined) | 6 | 0 | 2 | 3 | 6 | 12 | 23 | | | | | | 0 | 0 | 0 | 31:01 = 0 | 68% | 67% |
| | # alleles/ pirche score | | 56 | 2 | 1 | 1 | 2 | 26 | | | | | | | | | | | |
| A32 alleles | Pirche scores (combined) | 3 | 11 | 18 | 23 | | | | | | | | | 8 | 3 | 11 | 32:01 = 11 | 0% | 0% |
| | # alleles/ pirche score | | 39 | 2 | 24 | | | | | | | | | | | | | | |
| **A33** alleles | **Pirche** scores **(combined)** | 6 | 0 | 2 | 3 | 4 | 6 | 23 | | | | | | 2 | 0 | 2 | 33:01 **= 2** | 67% | 67% |
| | # **alleles/ pirche** score | | 12 | 43 | 1 | 1 | 1 | 29 | | | | | | | | | | | |
| A34 alleles | Pirche scores (combined) | 2 | 20 | 23 | | | | | | | | | | 14 | 6 | 23 | 34:01 = 20 | 0% | 0% |
| | # alleles/ pirche score | | 9 | 2 | | | | | | | | | | | | | | | |
| A36 alleles | Pirche scores (combined) | 1 | 19 | | | | | | | | | | | 13 | 6 | 19 | | 0% | 0% |
| | # alleles/ pirche score | | 5 | | | | | | | | | | | | | | | | |
| A43 alleles | Pirche scores (combined) | 1 | 20 | | | | | | | | | | | 14 | 6 | 20 | | 0% | 0% |
| | # alleles/ pirche score | | 1 | | | | | | | | | | | | | | | | |
| A66 alleles | Pirche scores (combined) | 2 | 20 | 23 | | | | | | | | | | 14 | 6 | 23 | 66:01 = 20 | 0% | 0% |
| | # alleles/ pirche score | | 16 | 3 | | | | | | | | | | | | | | | |
| A68 alleles | Pirche scores (combined) | 3 | 17 | 20 | 23 | | | | | | | | | 17 | 6 | 23 | 68:01 = 23 | 0% | 0% |
| | # alleles/ pirche score | | 1 | 1 | 110 | | | | | | | | | | | | | | |
| A69 alleles | Pirche scores (combined) | 1 | 23 | | | | | | | | | | | 17 | 6 | 23 | 69:01 = 23 | 0% | 0% |
| | # alleles/ pirche score | | 3 | | | | | | | | | | | | | | | | |
| A74 alleles | Pirche scores (combined) | 4 | 11 | 13 | 18 | 23 | | | | | | | | 8 | 3 | 11 | 74:01 = 11 | 0% | 0% |
| | # alleles/ pirche score | | 12 | 1 | 2 | 7 | | | | | | | | | | | | | |
| A80 alleles | Pirche scores (combined) | 2 | 21 | 23 | | | | | | | | | | 17 | 6 | 23 | 80:01 = 23 | 0% | 0% |
| | # alleles/ pirche score | | 1 | 2 | | | | | | | | | | | | | | | |
| | | | | | | | | | | | | | | | | | | | |

| **Pat-ient 2** | **A*02:01** | | **B*51:01** | **C*03:04** | **DRB1*14:54** | **DQB1*05:03** | | | | | | | | **Median PIRCHE score** | | | | | |
|---|---|---|---|---|---|---|---|---|---|---|---|---|---|---|---|---|---|---|---|
| | **A*01:01** | | B*15:01 | **C*15:02** | - | - | | | | | | | | | | | | | |
| | | Total # Pirche score groups | # Pirche peptides | # Pirche pepti-des | # Pirche peptides | # Pirche peptides | # Pir-che pepti-des | # Pir-che pepti-des | # Pir-che pepti-des | # Pir-che pepti-des | # Pir-che pepti-des | # Pir-che pepti-des | # Pirche peptides | I | II | I & II | | % al-leles <11 | % al-leles <6 |
| A1 al-leles | Pirche scores (combined) | 7 | 0 | 1 | 2 | 6 | 7 | 10 | 14 | | | | | 1 | 0 | 1 | | 66% | 63% |
| | # alleles/ pirche score | | 62 | 23 | 12 | 1 | 3 | 1 | 53 | | | | | | | | | | |
| A2 al-leles | Pirche scores (combined) | 2 | 12 | 14 | | | | | | | | | | 10 | 4 | 14 | A*02:0 1 = 14 | 0% | 0% |
| | # alleles/ pirche score | | 2 | 504 | | | | | | | | | | | | | | | |
| **A3 al-**leles | Pirche scores **(combined)** | 9 | 2 | **3** | 4 | **5** | 7 | 9 | 10 | 11 | 14 | | | 3 | 0 | 3 | 03:01 = 3 | 75% | 73% |
| | # alleles/ pirche score | | 4 | 120 | 3 | 10 | 1 | 2 | 1 | 2 | 45 | | | | | | | | |
| A11 alleles | Pirche scores **(combined)** | 9 | 1 | 2 | 3 | 4 | 5 | 7 | 8 | 11 | 14 | | | 2 | 0 | 4 | 11:01 = 2 | 61% | 59% |
| | # alleles/ pirche score | | 1 | 88 | 6 | 18 | 1 | 1 | 2 | 3 | 72 | | | | | | | | |
| A23 alleles | Pirche scores **(combined)** | 3 | 9 | 11 | 14 | | | | | | | | | 5 | 4 | 9 | 23:01 = 9 | 70% | 0% |
| | # alleles/ pirche score | | 44 | 3 | 16 | | | | | | | | | | | | | | |
| **A24 alleles** | **Pirche scores (combined)** | **4** | **15** | **19** | **21** | **23** | | | | | | | | **15** | **6** | **21** | **24:01 = 23, 24:02 = 21** | **0%** | **0%** |
| | # alleles/ pirche score | | 1 | 1 | 174 | 105 | | | | | | | | | | | | | |
| A25 alleles | Pirche scores (combined) | 2 | 13 | 14 | | | | | | | | | | 9 | 4 | 13 | 25:01 = 13 | 0% | 0% |
| | **#** alleles/ pirche score | | 15 | 10 | | | | | | | | | | | | | | | |
| A26 alleles | Pirche scores (combined) | 2 | 13 | 14 | | | | | | | | | | 9 | 4 | 13 | 26:01 = 13 | 0% | 0% |
| | **#** alleles/ pirche score | | 64 | 38 | | | | | | | | | | | | | | | |
| A29 alleles | Pirche scores (combined) | 3 | 10 | 12 | 14 | | | | | | | | | 8 | 3 | 11 | 29:01 = 10 | 50% | 0% |
| | **#** alleles/ pirche score | | 33 | 2 | 31 | | | | | | | | | | | | | | |
| **A30** alleles | **Pirche** scores **(combined)** | **6** | **3** | **5** | **7** | **9** | **11** | **14** | | | | | | **3** | **2** | **5** | **30:01 = 5** | **69%** | **65%** |
| | # **alleles/ pirche** score | | 1 | **54** | **3** | **1** | **2** | **24** | | | | | | | | | | | |
| **A31 alleles** | **Pirche scores (combined)** | **5** | **7** | **8** | **10** | **12** | **14** | | | | | | | **6** | **4** | **10** | **31:01 = 10** | **66%** | **0%** |
| | # **alleles/ pirche** score | | 1 | **2** | **55** | **4** | **26** | | | | | | | | | | | | |
| A32 alleles | Pirche scores (combined) | 3 | 10 | 12 | 14 | | | | | | | | | 8 | 4 | 12 | A*32:0 1 = 12 | 3% | 0% |
| | # alleles/ pirche score | | 2 | 39 | 24 | | | | | | | | | | | | | | |
| A33 alleles | Pirche scores (combined) | 5 | 8 | 10 | 11 | 12 | 14 | | | | | | | 8 | 4 | 12 | 33:01 = 12 | 17% | 0% |
| | **#** alleles/ pirche score | | 1 | 14 | 1 | 42 | 29 | | | | | | | | | | | | |
| A34 alleles | Pirche scores (combined) | 4 | 11 | 12 | 13 | 14 | | | | | | | | 9 | 2 | 12 | 34:01 = 13 | 0% | 0% |
| | **#** alleles/ pirche score | | 5 | 1 | 3 | 2 | | | | | | | | | | | | | |
| **A36 alleles** | **Pirche scores (combined)** | **2** | **1** | **2** | | | | | | | | | | **1** | **0** | **1** | **36:01 = 1** | **100%** | **100%** |
| | **# alleles/ pirche score** | | **4** | **1** | | | | | | | | | | | | | | | |
| A43 alleles | Pirche scores (combined) | 1 | 13 | | | | | | | | | | | 9 | 4 | 13 | | 0% | 0% |
| | **#** alleles/ pirche score | | 1 | | | | | | | | | | | | | | | | |
| A66 alleles | Pirche scores (combined) | 2 | 13 | 14 | | | | | | | | | | 9 | 4 | 13 | 66:01 = 13 | 0% | 0% |
| | **#** alleles/ pirche score | | 11 | 8 | | | | | | | | | | | | | | | |
| A68 alleles | Pirche scores (combined) | 3 | 12 | 13 | 14 | | | | | | | | | 10 | 3 | 13 | 68:01 = 12 | 0% | 0% |
| | **#** alleles/ pirche score | | 53 | 4 | 55 | | | | | | | | | | | | | | |
| A69 alleles | Pirche scores (combined) | 1 | 14 | | | | | | | | | | | 10 | 4 | 14 | 69:01 =14 | 0% | 0% |
| | **#** alleles/ pirche score | | 3 | | | | | | | | | | | | | | | | |
| A74 alleles | Pirche scores (combined) | 3 | 11 | 12 | 14 | | | | | | | | | 8 | 4 | 12 | 74:01 = 12 | 0% | 0% |
| | # alleles/ pirche score | | 1 | 14 | 7 | | | | | | | | | | | | | | |
| **A80 alleles** | **Pirche scores (combined)** | **3** | **6** | **10** | **14** | | | | | | | | | **8** | **2** | **10** | **80:01 = 10** | **67%** | **0%** |
| | **# alleles/ pirche score** | | **1** | **1** | **1** | | | | | | | | | | | | | | |
| | | | | | | | | | | | | | | | | | | | |

| **Pati-ent 3** | **A*02:01** | | **B*51:01** | **C*03:04** | **DRB1*14:54** | **DQB1*05:03** | | | | | | | | **Median PIRCHE score** | | | | | |
|---|---|---|---|---|---|---|---|---|---|---|---|---|---|---|---|---|---|---|---|
| | **A*23:01** | | **B*15:01** | **C*15:02** | - | - | | | | | | | | | | | | | |
| | | Total **#** Pirche score groups | **#** Pirche pepti-des | # Pirche pepti- \-des | **#** Pirche peptides | **#** Pirche peptides | **#** Pir- che pepti-des | **#** Pir- che pepti-des | **#** Pir-che pepti-des | **#** Pir-che pepti-des | **#** Pir-che pepti-des | **#** Pir-che pepti-des | **#** Pir- che peptides | I | II | I & II | | % al-leles <11 | % al-leles <6 |
| A1 al-leles | Pirche scores (combined) | 7 | 8 | 11 | 12 | 13 | 14 | 15 | 18 | | | | | 8 | 5 | 13 | 01:01 = 13 | 1% | 0% |
| | **#** alleles/ pirche score | | 2 | 3 | 1 | 87 | 3 | 2 | 57 | | | | | | | | | | |
| A2 al-leles | Pirche scores (combined) | 4 | 13 | 16 | 17 | 18 | | | | | | | | 13 | 5 | 18 | 02:01 = 18 | 0% | 0% |
| | **#** alleles/ pirche score | | 2 | 15 | 13 | 476 | | | | | | | | | | | | | |
| A3 al-leles | Pirche scores (combined) | 7 | 8 | 11 | 12 | 13 | 15 | 16 | 18 | | | | | 8 | 5 | 13 | 03:01 = 13 | 1% | 0% |
| | **#** alleles/ pirche score | | 1 | 1 | 1 | 133 | 1 | 1 | 50 | | | | | | | | | | |
| A11 alleles | Pirche scores (combined) | 6 | 8 | 11 | 13 | 14 | 15 | 18 | | | | | | 8 | 5 | 13 | 11:01 = 13 | 1% | 0% |
| | **#** alleles/ pirche score | | 1 | 1 | 99 | 8 | 5 | 78 | | | | | | | | | | | |
| A23 alleles | Pirche scores (combined) | 10 | 0 | 1 | 2 | 3 | 4 | 5 | 7 | 9 | 13 | 18 | | 0 | 0 | 1 | 23:01 = 0 | 78% | 75% |
| | # alleles/ pirche score | | 26 | 11 | 7 | 1 | 1 | 1 | 1 | 1 | 2 | 12 | | | | | | | |
| **A24 alleles** | **Pirche scores (combined)** | **11** | **2** | **3** | **4** | **5** | **7** | **8** | **9** | **10** | **11** | **13** | **18** | **3** | **1** | **5** | **24:01 = 18, 24:02 = 3** | **66%** | **61%** |
| | **# alleles/ pirche score** | | **1** | **108** | **28** | **34** | **4** | **8** | **1** | **2** | **2** | **3** | **90** | | | | | | |
| A25 alleles | Pirche scores (combined) | 1 | 18 | | | | | | | | | | | 13 | 5 | 18 | | 0% | 0% |
| | **#** alleles/ pirche score | | 25 | | | | | | | | | | | | | | | | |
| A26 alleles | Pirche scores (combined) | 2 | 13 | 18 | | | | | | | | | | 13 | 5 | 18 | 26:01 = 18 | 0% | 0% |
| | **#** alleles/ pirche score | | 1 | 101 | | | | | | | | | | | | | | | |
| A29 alleles | Pirche scores (combined) | 3 | 15 | 16 | 18 | | | | | | | | | 13 | 5 | 18 | 29:01 = 18 | 0% | 0% |
| | **#** alleles/ pirche score | | 1 | 1 | 64 | | | | | | | | | | | | | | |
| **A30 alleles** | **Pirche scores (combined)** | **6** | **6** | **8** | **10** | **12** | **13** | **18** | | | | | | **5** | **3** | **8** | **30:01 = 8** | **65%** | **0%** |
| | **# alleles/ pirche score** | | **1** | **51** | **3** | **1** | **5** | **24** | | | | | | | | | | | |
| A31 alleles | Pirche scores (combined) | 5 | 13 | 14 | 15 | 16 | 18 | | | | | | | 11 | 5 | 16 | 31:01 = 16 | 0% | 0% |
| | **#** alleles/ pirche score | | 3 | 5 | 3 | 47 | 30 | | | | | | | | | | | | |
| A32 alleles | Pirche scores (combined) | 5 | 13 | 15 | 16 | 17 | 18 | | | | | | | 13 | 5 | 18 | 32:01 = 18 | 0% | 0% |
| | **#** alleles/ pirche score | | 1 | 3 | 2 | 2 | 57 | | | | | | | | | | | | |
| A33 alleles | Pirche scores (combined) | 4 | 11 | 15 | 16 | 18 | | | | | | | | 13 | 5 | 18 | 33:01 = 16 | 0% | 0% |
| | **#** alleles/ pirche score | | 1 | 1 | 24 | 18 | | | | | | | | | | | | | |
| A34 alleles | Pirche scores (combined) | 1 | 18 | | | | | | | | | | | 13 | 5 | 18 | | 0% | 0% |
| | **#** alleles/ pirche score | | 11 | | | | | | | | | | | | | | | | |
| A36 alleles | Pirche scores (combined) | 1 | 13 | | | | | | | | | | | 8 | 5 | 13 | | 0% | 0% |
| | **#** alleles/ pirche score | | 5 | | | | | | | | | | | | | | | | |
| A43 alleles | Pirche scores (combined) | 1 | 18 | | | | | | | | | | | 13 | 5 | 18 | | 0% | 0% |
| | **#** alleles/ pirche score | | 1 | | | | | | | | | | | | | | | | |
| A66 alleles | Pirche scores (combined) | 1 | 18 | | | | | | | | | | | 13 | 5 | 18 | | 0% | 0% |
| | **#** alleles/ pirche score | | 19 | | | | | | | | | | | | | | | | |
| A68 alleles | Pirche scores (combined) | 5 | 13 | 15 | 16 | 17 | 18 | | | | | | | 13 | 5 | 18 | 68:01 = 18 | 0% | 0% |
| | **#** alleles/ pirche score | | 1 | 1 | 2 | 24 | 84 | | | | | | | | | | | | |
| A69 alleles | Pirche scores (combined) | 1 | 18 | | | | | | | | | | | 13 | 5 | 18 | 69:01 = 18 | 0% | 0% |
| | **#** alleles/ pirche score | | 3 | | | | | | | | | | | | | | | | |
| A74 alleles | Pirche scores (combined) | 1 | 18 | | | | | | | | | | | 13 | 5 | 18 | | 0% | 0% |
| | **#** alleles/ pirche score | | 22 | | | | | | | | | | | | | | | | |
| A80 alleles | Pirche scores (combined) | 3 | 13 | 16 | 18 | | | | | | | | | 11 | 5 | 16 | 80:01 = 16 | 0% | 0% |
| | **#** alleles/ pirche score | | 1 | 1 | 1 | | | | | | | | | | | | | | |
| | | | | | | | | | | | | | | | | | | | |

| **Pati-ent 4** | **A*02:01** | | **B*51:01** | **C*3:04** | **DRB1*14:54** | **DQB1*05:03** | | | | | | | | **Median PIRCHE score** | | | | | |
|---|---|---|---|---|---|---|---|---|---|---|---|---|---|---|---|---|---|---|---|
| | **A*11:01** | | **B*15:01** | **C*15:02** | - | - | | | | | | | | | | | | | |
| | | Total **#** Pirche score groups | **#** Pirche peptides | **#** Pirche pepti-des | **#** Pirche peptides | **#** Pirche peptides | **#** Pir- che pepti-des | **#** Pir-che pepti-des | **#** Pir-che pepti-des | **#** Pir-che pepti-des | **#** Pir-che pepti-des | **#** Pir-che pepti-des | **#** Pir- che peptides | I | II | I & II | | % al-leles <11 | % al-leles <6 |
| **A1 al-leles** | **Pirche scores (combined)** | **7** | **0** | **5** | **6** | **7** | **11** | **14** | **17** | | | | | **3** | **2** | **6** | | **63%** | **49%** |
| | **# alleles/ pirche score** | | **1** | **75** | **11** | **11** | **6** | **1** | **50** | | | | | | | | | | |
| A2 al-leles | Pirche scores (combined) | 4 | 12 | 13 | 15 | 17 | | | | | | | | 11 | 6 | 17 | 02:01 = 17 | 0% | 0% |
| | **#** alleles/ pirche score | | 50 | 3 | 2 | 451 | | | | | | | | | | | | | |
| **A3 al-leles** | **Pirche scores (combined)** | **11** | **0** | **1** | **5** | **6** | **7** | **8** | **10** | **12** | **13** | **14** | **17** | **4** | **2** | **6** | | **73%** | **2%** |
| | **# alleles/ pirche score** | | **1** | **1** | **2** | **120** | **3** | **10** | **1** | **2** | **1** | **2** | **45** | | | | | | |
| A11 alleles | Pirche scores (combined) | 8 | 0 | 1 | 2 | 3 | 5 | 6 | 9 | 17 | | | | 1 | 1 | 2 | | 63% | 60% |
| | **#** alleles/ pirche score | | 81 | 8 | 18 | 1 | 7 | 2 | 3 | 72 | | | | | | | | | |
| A23 alleles | Pirche scores (combined) | 3 | 12 | 14 | 17 | | | | | | | | | 6 | 6 | 12 | 23:01 = 12 | 0% | 0% |
| | **#** alleles/ pirche score | | 44 | 3 | 16 | | | | | | | | | | | | | | |
| A24 alleles | Pirche scores (combined) | 9 | 7 | 9 | 10 | 12 | 13 | 14 | 15 | 16 | 17 | | | 6 | 6 | 12 | 24:01 = 17 | 1% | 0% |
| | **#** alleles/ pirche score | | 1 | 1 | 2 | 167 | 4 | 5 | 1 | 1 | 99 | | | | | | | | |
| A25 alleles | Pirche scores (combined) | 4 | 11 | 12 | 16 | 17 | | | | | | | | 7 | 4 | 11 | 25:01 = 11 | 0% | 0% |
| | **#** alleles/ pirche score | | 14 | 1 | 1 | 9 | | | | | | | | | | | | | |
| A26 alleles | Pirche scores (combined) | 4 | 11 | 12 | 16 | 17 | | | | | | | | 7 | 4 | 11 | 26:01 = 11 | 0% | 0% |
| | **#** alleles/ pirche score | | 61 | 8 | 3 | 30 | | | | | | | | | | | | | |
| A29 alleles | Pirche scores (combined) | 3 | 13 | 15 | 17 | | | | | | | | | 9 | 5 | 14 | 29:01 = 13 | 0% | 0% |
| | **#** alleles/ pirche score | | 33 | 2 | 31 | | | | | | | | | | | | | | |
| **A30 alleles** | **Pirche scores (combined)** | **7** | **3** | **6** | **8** | **10** | **12** | **14** | **17** | | | | | **4** | **4** | **8** | **30:01 = 8** | **68%** | **1%** |
| | **# alleles/ pirche score** | | **1** | **1** | **53** | **3** | **1** | **2** | **24** | | | | | | | | | | |
| A31 alleles | Pirche scores (combined) | 5 | 10 | 11 | 13 | 15 | 17 | | | | | | | 7 | 6 | 13 | 31:01 = 13 | 1% | 0% |
| | **#** alleles/ pirche score | | 1 | 2 | 55 | 4 | 26 | | | | | | | | | | | | |
| A32 alleles | Pirche scores (combined) | 3 | 13 | 15 | 17 | | | | | | | | | 9 | 6 | 15 | 32:01 = 15 | 0% | 0% |
| | **#** alleles/ pirche score | | 2 | 39 | 24 | | | | | | | | | | | | | | |
| A33 alleles | Pirche scores (combined) | 6 | 8 | 11 | 13 | 14 | 15 | 17 | | | | | | 9 | 6 | 15 | 33:01 = 15 | 1% | 0% |
| | **#** alleles/ pirche score | | 1 | 1 | 13 | 1 | 42 | 29 | | | | | | | | | | | |
| A34 alleles | Pirche scores (combined) | 5 | 9 | 10 | 11 | 14 | 17 | | | | | | | 7 | 4 | 11 | 34:01 = 11 | 45% | 0% |
| | **#** alleles/ pirche score | | 4 | 1 | 3 | 1 | 2 | | | | | | | | | | | | |
| **A36 alleles** | **Pirche scores (combined)** | **2** | **5** | **6** | | | | | | | | | | **3** | **2** | **5** | | **100%** | **80%** |
| | **# alleles/ pirche score** | | **4** | **1** | | | | | | | | | | | | | | | |
| A43 alleles | Pirche scores (combined) | 1 | 11 | | | | | | | | | | | 7 | 4 | 11 | | 0% | 0% |
| | **#** alleles/ pirche score | | 1 | | | | | | | | | | | | | | | | |
| A66 alleles | Pirche scores (combined) | 3 | 11 | 12 | 17 | | | | | | | | | 7 | 4 | 11 | 66:01 = 11 | 0% | 0% |
| | **#** alleles/ pirche score | | 11 | 5 | 3 | | | | | | | | | | | | | | |
| A68 alleles | Pirche scores (combined) | 6 | 10 | 11 | 12 | 14 | 15 | 17 | | | | | | 9,5 | 4 | 13 | 68:01 =10 | 43% | 0% |
| | **#** alleles/ pirche score | | 48 | 4 | 4 | 1 | 4 | 51 | | | | | | | | | | | |
| A69 alleles | Pirche scores (combined) | 2 | 12 | 17 | | | | | | | | | | 11 | 6 | 17 | 69:01 = 12 | 0% | 0% |
| | **#** alleles/ pirche score | | 1 | 2 | | | | | | | | | | | | | | | |
| A74 alleles | Pirche scores (combined) | 3 | 14 | 15 | 17 | | | | | | | | | 9 | 6 | 15 | 74:01 =15 | 0% | 0% |
| | **#** alleles/ pirche score | | 1 | 14 | 7 | | | | | | | | | | | | | | |
| A80 alleles | Pirche scores (combined) | 3 | 9 | 13 | 17 | | | | | | | | | 9 | 4 | 13 | | 33% | 0% |
| | **#** alleles/ pirche score | | 1 | 1 | 1 | | | | | | | | | | | | | | |

**Table 3.**

| **Pati-ent 1** | **A*02:01** | | **B*51:01** | **C*03:04** | **DRB1*14:54** | **DQB1*05:03** | | | | | | | | **Median PIRCHE score** | | | | | |
|---|---|---|---|---|---|---|---|---|---|---|---|---|---|---|---|---|---|---|---|
| | **A*31:01** | | **B*15:01** | **C*****15:02** | - | - | | | | | | | | | | | | | |
| | | Total **#** Pirche score groups | **#** Pirche pepti-des | **#** Pirche pepti-des | **#** Pirche peptides | **#** Pirche peptides | **#** Pir- che pepti-des | **#** Pir- che pepti-des | **#** Pir-che pepti-des | **#** Pir-che pepti-des | **#** Pir-che pepti-des | **#** Pir-che pepti-des | **#** Pir-che pepti-des | I | II | I & II | | % al-leles <11 | % alle-les <6 |
| A31 alleles | Pirche scores (combined) | 6 | 0 | 2 | 3 | 6 | 12 | 23 | | | | | | 0 | 0 | 0 | 31:01 = 0 | 68% | 67% |
| | # alleles/ pirche score | | 56 | 2 | 1 | 1 | 2 | 26 | | | | | | | | | | | |
| **A29 alle-les** | **Pirche scores (combi--ned)** | **3** | **2** | **12** | **23** | | | | | | | | | **2** | **0** | **2** | **29:01 = 2** | **52%** | **52%** |
| | **#** alleles/ pirche score | | 34 | 1 | 31 | | | | | | | | | | | | | | |
| **A33 alle-les** | **Pirche scores (combi-ned)** | **6** | **0** | **2** | **3** | **4** | **6** | **23** | | | | | | **2** | **0** | **2** | **33:01 = 2** | **67%** | **67%** |
| | **#** alleles/ pirche score | | 12 | 43 | 1 | 1 | 1 | 29 | | | | | | | | | | | |
| | | | | | | | | | | | | | | | | | | | |
| **Pati-ent 2** | **A*02:01** | | **B*51:01** | **C*03:04** | **DRB1*14:54** | **DQB1*05:03** | | | | | | | | **Median PIRCHE score** | | | | | |
| | **A*01:01** | | **B*15:01** | **C*15:02** | - | - | | | | | | | | | | | | | |
| | | Total **#** Pirche score groups | **#** Pirche pepti-des | **#** Pirche pepti-des | **#** Pirche peptides | **#** Pirche peptides | **#** Pir-che pepti-des | **#** Pir-che pepti-des | **#** Pir-che pepti-des | **#** Pir-che pepti-des | **#** Pir-che pepti-des | **#** Pir-che pepti-des | **#** Pir-che pepti-des | I | II | I & II | | | |
| A1 al-leles | Pirche scores (combined) | 7 | 0 | 1 | 2 | 6 | 7 | 10 | 14 | | | | | 1 | 0 | 1 | | 66% | 63% |
| | **#** alleles/ pirche score | | 62 | 23 | 12 | 1 | 3 | 1 | 53 | | | | | | | | | | |
| **A3 al-leles** | **Pirche scores (combi-ned)** | **9** | **2** | **3** | **4** | **5** | **7** | **9** | **10** | **11** | **14** | | | **3** | **0** | **3** | **03:01 = 3** | **75%** | **73%** |
| | **#** alleles/ pirche score | | 4 | 120 | 3 | 10 | 1 | 2 | 1 | 2 | 45 | | | | | | | | |
| **A11 alle-les** | **Pirche scores (combi-ned)** | **9** | **1** | **2** | **3** | **4** | **5** | **7** | **8** | **11** | **14** | | | **2** | **0** | **4** | **11:01 = 2** | **61%** | **59%** |
| | **#** alleles/ pirche score | | 1 | 88 | 6 | 18 | 1 | 1 | 2 | 3 | 72 | | | | | | | | |
| **A30 alle-les** | **Pirche scores (combi-ned)** | **6** | **3** | **5** | **7** | **9** | **11** | **14** | | | | | | **3** | **2** | **5** | **30:01 = 5** | **69%** | **65%** |
| | **#** alleles/ pirche score | | 1 | 54 | 3 | 1 | 2 | 24 | | | | | | | | | | | |
| **A36 alle-**les | **Pirche scores (combi-ned)** | **2** | **1** | **2** | | | | | | | | | | **1** | **0** | **1** | **36:01 = 1** | **100%** | **####** |
| | # alleles/ pirche score | | 4 | 1 | | | | | | | | | | | | | | | |
| | | | | | | | | | | | | | | | | | | | |

| **Pati-ent 3** | **A*02:01** | | B*51:01 | **C*03:04** | **DRB1*14:54 DQB1*14:54** | **DQB1*05:03** | | | | | | | | **Median PIRCHE score** | | | | | |
|---|---|---|---|---|---|---|---|---|---|---|---|---|---|---|---|---|---|---|---|
| | **A*23:01** | | **B*15:01** | **C*15:02** - | - | - | | | | | | | | | | | | | |
| | | Total **#** Pirche score groups | **#** Pirche pepti-des | **#** Pirche pepti-des | **#** Pirche peptides | **#** Pirche peptides | **#** Pir-che pepti-des | **#** Pir-che pepti-des | **#** Pir-che pepti-des | **#** Pir-che pepti-des | **#** Pir-che pepti-des | **#** Pir-che pepti-des | **#** Pir-che pepti-des | I | II | I & II | | | |
| A23 alleles | Pirche scores (combined) | 10 | 0 | 1 | 2 | 3 | 4 | 5 | 7 | 9 | 13 | 18 | | 0 | 0 | 1 | 23:01 = 0 | 78% | 75% |
| | **#** alleles/ pirche score | | 26 | 11 | 7 | 1 | 1 | 1 | 1 | 1 | 2 | 12 | | | | | | | |
| **A24 alle-les** | **Pirche scores (combi-ned)** | **11** | **2** | **3** | **4** | **5** | **7** | **8** | **9** | **10** | **11** | **13** | **18** | **3** | **1** | **5** | **24:01 = 18, 24:02 = 3** | **66%** | **61%** |
| | **#** alleles/ pirche score | | 1 | 108 | 28 | 34 | 4 | 8 | 1 | 2 | 2 | 3 | 90 | | | | | | |
| | | | | | | | | | | | | | | | | | | | |

| **Patient 4** | **A*02:01** | | **B*51:01** | **C*03:04** | **DRB1*14:54** | **DQB1*05:03** | | | | | | | | **Median PIRCHE score** | | | | | |
|---|---|---|---|---|---|---|---|---|---|---|---|---|---|---|---|---|---|---|---|
| | **A*11:01 B*15:01 C*15:02** | | | | - | - | | | | | | | | | | | | | |
| | | Total **#** Pirche score groups | **#** Pirche pepti-des | **#** Pirche pepti-des | **#** Pirche peptides | **#** Pirche peptides | **#** Pir- che pepti-des | **#** Pir- che pepti-des | **#** Pir-che pepti-des | **#** Pir-che pepti-des | **#** Pir-che pepti-des | **#** Pir-che pepti-des | **#** Pir- che peptides | I | II | I & II | | | |
| A11 alleles | Pirche scores (combined) | 8 | 0 | 1 | 2 | 3 | 5 | 6 | 9 | 17 | | | | 1 | 1 | 2 | | 63% | 60% |
| | **#** alleles/ pirche score | | 81 | 8 | 18 | 1 | 7 | 2 | 3 | 72 | | | | | | | | | |
| **A1 al-leles** | **Pirche scores (combined)** | **7** | **0** | **5** | **6** | **7** | **11** | **14** | **17** | | | | | **3** | **2** | **6** | | **63%** | **49%** |
| | # alleles/ pirche score | | 1 | 75 | 11 | 11 | 6 | 1 | 50 | | | | | | | | | | |
| **A36 alle-les** | **Pirche scores (combined)** | **2** | **5** | **6** | | | | | | | | | | **3** | **2** | **5** | | **100%** | **80%** |
| | **#** alleles/ pirche score | | 4 | 1 | | | | | | | | | | | | | | | |

## Claims

1. Method for selecting a donor for providing transplantation material, comprising:
a. Providing an electronic representation of HLA-typing data from a transplantation recipient and one or more candidate transplantation donors, wherein two-field specific HLA protein typing is unavailable for said donor(s) and/or recipient,
b. Determining in a computer-implemented method the number of predicted indirectly recognized HLA epitopes (PIRCHES) (PIRCHE value) for multiple specific HLA proteins of the donor allele group of the HLA locus that is mismatched or potentially mismatched between said donor and said recipient (mismatched donor allele group); using the data provided in step (a) and
c. Selecting or rejecting one or more of said candidate transplantation donors in a computer-implemented method according to the PIRCHE values determined in step b).

2. Method for selecting a mismatched allele group of a transplantation donor, comprising:
a. Providing an electronic representation of HLA-typing data from a transplantation recipient and one or more candidate transplantation donors, wherein two-field specific HLA protein typing is unavailable for said donor(s) and/or recipient,
b. Determining in a computer-implemented method the number of predicted indirectly recognized HLA epitopes (PIRCHES) (PIRCHE value) for multiple specific HLA proteins of multiple allele groups of a mismatched allele between said donor and said recipient; using the data provided in step (a) and
c. Selecting or rejecting one or more mismatched allele groups of a transplantation donor in a computer-implemented method according to the PIRCHE values determined in step b).

3. Method according to claim 1, wherein at least one HLA mismatch (at any HLA loci) is present between said donor(s) and said recipient at the allele group level.

4. Method according to any one of the preceding claims, wherein specific HLA protein typing information is available for said recipient and specific HLA protein typing information is not available for said donor, or wherein two-field specific HLA protein typing is unavailable and HLA allele group typing information is available for said recipient.

5. Method according to any one of the preceding claims, wherein said transplantation donor or mismatched allele group of a transplantation donor is associated with reduced risk of an unwanted immune response against human leukocyte antigens (HLA) after transplantation.

6. Method according to any one of the preceding claims, wherein the PIRCHE value is the sum of PIRCHE-I and PIRCHE-II values.

7. Method according to any one of the preceding claims, wherein more than one candidate transplantation donors are provided, and wherein the likelihood (risk) of an unwanted immune response against human leukocyte antigens (HLA) in the recipient after transplantation is lower for transplantation material obtained from donors with a relatively greater proportion of specific-HLA proteins of the mismatched donor allele group that exhibit relatively lower PIRCHE values compared to other candidate donors, or PIRCHE values less than or equal to a pre-set value (defined by the user), compared to other candidate donors.

8. Method according to any one of the preceding claims, comprising:
a. determining the distribution of PIRCHE values of specific HLA proteins of the mismatched donor allele group, wherein a candidate transplantation donor is selected for providing transplantation material when a relatively greater proportion of specific-HLA proteins of the mismatched donor allele group exhibit relatively lower PIRCHE values compared to other candidate donors,
b. determining the median PIRCHE value of the specific HLA proteins of the mismatched donor allele group, wherein a candidate transplantation donor is selected for providing transplantation material when the median PIRCHE value is less than or equal to a pre-set value, preferably wherein a candidate transplantation donor is selected for providing transplantation material when the median PIRCHE value is less than or equal to 10, more preferably less than or equal to 5, and optionally determining the median PIRCHE value and standard deviation of the specific HLA proteins of the mismatched donor allele group, or
c. determining the percentage of the specific HLA proteins of the mismatched donor allele group that have a PIRCHE value less than or equal to a pre-set value, wherein a candidate transplantation donor is selected for providing transplantation material when greater than the pre-set percentage of the specific HLA proteins of the mismatched donor allele group have a PIRCHE value less than or equal to a pre-set value, preferably wherein the pre-set percentage is 50% and/or the pre-set value is less than or equal to 10, more preferably less than or equal to 5.

9. Method according to any one of the preceding claims, wherein the candidate transplantation donor is selected for providing transplantation material for a biological transplantation procedure, wherein the biological transplantation is an organ transplantation or a cell transplantation, preferably a stem cell transplantation, such as a hematopoietic stem cell (HSC) transplantation, for example wherein said HSC are obtained from cord blood.

10. Method according to any one of the preceding claims, wherein one or more steps of the method are carried out in a computer implemented method, wherein
a. providing a candidate transplantation donor(s) and transplantation recipient comprises electronic (in silico) representation and/or processing of the HLA typing information available for said donor and said recipient in a computer-implemented method,
b. the sequences of specific HLA proteins of any given mismatched donor allele group are provided, obtained and/or processed as an electronic (in silico) representation of HLA protein sequences of any given HLA locus,
c. the electronic (in silico) representation of HLA protein sequences of any given HLA locus is stored in a databank comprising multiple known HLA allele sequences, and/or
d. candidate donors are electronically (in silico) generated to represent every possible mismatched specific HLA protein combination to the transplantation recipient in a computer-implemented method.

11. Method according to any one of the preceding claims, comprising determining the PIRCHE value for multiple specific HLA protein of multiple allele groups of a mismatched allele for multiple mismatched HLA loci, thereby identifying a mismatched HLA locus associated with relatively low PIRCHE values compared to other mismatched HLA loci.

12. Method according to any one of the preceding claims, comprising processing information representing the frequency of any given specific HLA protein and/or HLA allele group (haplotype or genotype) in any one or more human populations, such as defined by age, gender and/or ethnicity, during selecting a donor or mismatched allele group.

13. Method for reducing the risk of an unwanted immune response against human leukocyte antigens (HLA) after transplantation in a recipient and/or for increasing the likelihood of obtaining a mismatched transplantation donor associated with a reduced risk of inducing said unwanted immune response comprising carrying out the method according to any one of the preceding claims.

14. Method according to any one of the preceding claims, for predicting an immune response against human leukocyte antigens (HLA) after transplantation, said method comprising:
a. determination of the number of predicted indirectly recognized HLA epitopes (PIRCHE), wherein said PIRCHE are recipient- or donor-specific HLA-derived peptides from the mismatched recipient-HLA allele and are predicted to be presented by a shared (matched) HLA molecule, for the selection of a transplantation donor for providing transplantation material, wherein HLA allele group typing information is available and HLA protein-specific typing information is unavailable for said donor, or
b.determination of the number of predicted indirectly recognized HLA epitopes (PIRCHE), wherein said PIRCHE are recipient- or donor-specific HLA-derived peptides from the mismatched donor-HLA allele and are predicted to be presented by a shared (matched) and non-shared (mismatched) HLA molecule, for the selection of a transplantation donor for providing transplantation material, wherein the HLA protein-specific typing information is unavailable for said donor and recipient.

15. System for selecting a transplantation donor for providing transplantation material, said system comprising computing devices, data storage devices and/or software as system components, wherein said components are configured to interact with one or more of other said components and to carry out the computer-implemented steps of the method according to any one of the preceding claims.

## Patentansprüche

1. Verfahren zum Auswählen eines Spenders zum Bereitstellen von Transplantationsmaterial, umfassend:
a. Bereitstellen einer elektronischen Darstellung von HLA-Typisierungsdaten eines Transplantationsempfängers und eines oder mehrerer Transplantationsspenderkandidaten, wobei für den/die Spender und/oder den Empfänger keine zweifeldspezifische HLA-Protein-Typisierung verfügbar ist,
b. Bestimmen der Anzahl der vorhergesagten indirekt erkannten HLA-Epitope (PIRCHES) (PIRCHE-Wert) für mehrere spezifische HLA-Proteine der Spender-Allelgruppe der HLA-Stelle, der zwischen dem Spender und dem Empfänger nicht übereinstimmt oder potenziell nicht übereinstimmt (Gruppe der nicht übereinstimmenden Spenderallele), in einem computerimplementierten Verfahren; Verwendung der in Schritt (a) bereitgestellten Daten und
c. Auswählen oder Ablehnen eines oder mehrerer der Transplantationsspenderkandidaten in einem computerimplementierten Verfahren gemäß den in Schritt b) bestimmten PIRCHE-Werten.

2. Verfahren zum Auswählen einer nicht übereinstimmenden Allelgruppe eines Transplantationsspenders, umfassend:
a. Bereitstellen einer elektronischen Darstellung von HLA-Typisierungsdaten von einem Transplantationsempfänger und einem oder mehreren Transplantationsspenderkandidaten, wobei eine zweifeldspezifischer HLA-Protein-Typisierung für den/die Spender und/oder Empfänger nicht verfügbar ist,
b. Bestimmen der Anzahl der vorhergesagten indirekt erkannten HLA-Epitope (PIRCHES) (PIRCHE-Wert) für mehrere spezifische HLA-Proteine mehrerer Allelgruppen eines nicht übereinstimmenden Allels zwischen dem Spender und dem Empfänger in einem computerimplementierten Verfahren; unter Verwendung der in Schritt (a) bereitgestellten Daten und
c. Auswählen oder Ablehnen einer oder mehrerer nicht übereinstimmender Allelgruppen eines Transplantationsspenders in einem computerimplementierten Verfahren gemäß den in Schritt b) bestimmten PIRCHE-Werten.

3. Verfahren gemäß Anspruch 1, wobei mindestens eine HLA-Nichtübereinstimmung (an beliebigen HLA-Stellen) zwischen dem/den Spender(n) und dem Empfänger auf der Allelgruppenebene vorliegt.

4. Verfahren gemäß einem der vorstehenden Ansprüche, wobei spezifische HLA-Protein-Typisierungsinformationen für den Empfänger verfügbar sind und spezifische HLA-Protein-Typisierungsinformationen für den Spender nicht verfügbar sind, oder wobei eine zweifeldspezifischer HLA-Protein-Typisierung nicht verfügbar ist und HLA-Allelgruppen-Typisierungsinformationen für den Empfänger verfügbar sind.

5. Verfahren gemäß einem der vorstehenden Ansprüche, wobei der Transplantationsspender oder die nicht übereinstimmende Allelgruppe eines Transplantationsspenders mit einem verringerten Risiko einer unerwünschten Immunreaktion gegen humane Leukozytenantigene (HLA) nach der Transplantation assoziiert ist.

6. Verfahren gemäß einem der vorstehenden Ansprüche, wobei der PIRCHE-Wert die Summe der PIRCHE-I und PIRCHE-II-Werte ist.

7. Verfahren gemäß einem der vorstehenden Ansprüche, wobei mehr als ein Transplantationsspenderkandidat bereitgestellt ist, und wobei die Wahrscheinlichkeit (das Risiko) einer unerwünschten Immunreaktion gegen humane Leukozytenantigene (HLA) im Empfänger nach der Transplantation geringer ist für Transplantationsmaterial, das von Spendern mit einem relativ größeren Anteil spezifischer HLA-Proteine der Gruppe der nicht übereinstimmenden Spenderallele erhalten wird, die im Vergleich zu anderen Spenderkandidaten relativ niedrigere PIRCHE-Werte oder PIRCHE-Werte aufweisen, die kleiner oder gleich einem voreingestellten (vom Benutzer definierten) Wert sind, verglichen mit anderen Spenderkandidaten.

8. Verfahren gemäß einem der vorstehenden Ansprüche, umfassend:
a. Bestimmen der Verteilung der PIRCHE-Werte spezifischer HLA-Proteine der Gruppe der nicht übereinstimmenden Spenderallele, wobei ein Transplantationsspenderkandidat für das Bereitstellen von Transplantationsmaterial ausgewählt wird, wenn ein relativ größerer Anteil spezifischer HLA-Proteine der Gruppe der nicht übereinstimmenden Spenderallele im Vergleich zu anderen Spenderkandidaten relativ niedrigere PIRCHE-Werte aufweist,
b. Bestimmen des mittleren PIRCHE-Wertes der spezifischen HLA-Proteine der Gruppe der nicht übereinstimmenden Spenderallele, wobei ein Transplantationsspenderkandidat zum Bereitstellen von Transplantationsmaterial ausgewählt wird, wenn der mittlere PIRCHE-Wert kleiner oder gleich einem voreingestellten Wert ist, wobei ein Transplantationsspenderkandidat für ein Bereitstellen von Transplantationsmaterial bevorzugt ausgewählt wird, wenn der mittlere PIRCHE-Wert kleiner oder gleich 10, noch bevorzugter kleiner oder gleich 5 ist, und optional ein Bestimmen des mittleren PIRCHE-Wertes und der Standardabweichung der spezifischen HLA-Proteine der Gruppe der nicht übereinstimmenden Spenderallele, oder
c. Bestimmen des Prozentsatzes der spezifischen HLA-Proteine der Gruppe der nicht übereinstimmenden Spenderallele, die einen PIRCHE-Wert aufweisen, der kleiner oder gleich einem vorgegebenen Wert ist, wobei ein Transplantationsspenderkandidat für ein Bereitstellen von Transplantationsmaterial ausgewählt wird, wenn mehr als der voreingestellte Prozentsatz der spezifischen HLA-Proteine der Gruppe der nicht übereinstimmenden Spenderallele einen PIRCHE-Wert aufweisen, der kleiner als oder gleich einem voreingestellten Wert ist, wobei der voreingestellte Prozentsatz bevorzugt 50 % beträgt und/oder der voreingestellte Wert kleiner als oder gleich 10 ist, noch bevorzugter kleiner als oder gleich 5.

9. Verfahren gemäß einem der vorstehenden Ansprüche, wobei der Transplantationsspenderkandidat für ein Bereitstellen von Transplantationsmaterial für ein biologisches Transplantationsverfahren ausgewählt wird, wobei die biologische Transplantation eine Organtransplantation oder eine Zelltransplantation, bevorzugt eine Stammzelltransplantation, wie eine hämatopoetische Stammzelltransplantation (HSC), ist, wobei die HSC beispielsweise aus Nabelschnurblut erhalten werden.

10. Verfahren gemäß einem der vorstehenden Ansprüche, wobei ein oder mehrere Schritte des Verfahrens in einem computerimplementierten Verfahren durchgeführt werden, wobei
a. ein Bereitstellen eines Transplantationsspenders und eines Transplantationsempfängerkandidaten eine elektronische (in silico) Darstellung und/oder Verarbeitung der für den Spender und den Empfänger verfügbaren Informationen zur HLA-Typisierung in einem computerimplementierten Verfahren umfasst,
b. die Sequenzen spezifischer HLA-Proteine jeder gegebenen Gruppe der nicht übereinstimmenden Spenderallele als eine elektronische (in silico) Darstellung von HLA-Proteinsequenzen jeder gegebenen HLA-Stelle bereitgestellt, erhalten und/oder verarbeitet werden,
c. die elektronische (in silico) Darstellung der HLA-Proteinsequenzen jeder gegebenen HLA-Stelle in einer Datenbank gespeichert wird, die mehrere bekannte HLA-Allelsequenzen umfasst, und/oder
d. Spenderkandidaten elektronisch (in silico) generiert werden, um jede mögliche nicht übereinstimmende spezifische HLA-Protein-Kombination für den Transplantationsempfänger in einem computerimplementierten Verfahren darzustellen.

11. Verfahren gemäß einem der vorstehenden Ansprüche, umfassend ein Bestimmen des PIRCHE-Wertes für mehrere spezifische HLA-Proteine mehrerer Allelgruppen eines nicht übereinstimmenden Allels für mehrere nicht übereinstimmende HLA-Stellen, wodurch eine nicht übereinstimmende HLA-Stelle identifiziert wird, die mit relativ niedrigen PIRCHE-Werten im Vergleich zu anderen nicht übereinstimmenden HLA-Stellen assoziiert ist.

12. Verfahren gemäß einem der vorstehenden Ansprüche, umfassend ein Verarbeiten von Informationen, die die Häufigkeit eines gegebenen spezifischen HLA-Proteins und/oder einer gegebenen HLA-Allelgruppe (Haplotyp oder Genotyp) in jedem oder mehreren menschlichen Populationen, z. B. definiert durch Alter, Geschlecht und/oder ethnische Zugehörigkeit, während der Auswahl eines Spenders oder einer Gruppe nicht übereinstimmender Allele darstellen.

13. Verfahren zum Verringern des Risikos einer unerwünschten Immunreaktion gegen menschliche Leukozytenantigene (HLA) nach einer Transplantation in einem Empfänger und/oder zum Erhöhen der Wahrscheinlichkeit, einen nicht übereinstimmenden Transplantationsspender zu erhalten, der mit einem verringerten Risiko der Induktion der unerwünschten Immunreaktion assoziiert ist, umfassend ein Durchführen des Verfahrens nach einem der vorstehenden Ansprüche.

14. Verfahren gemäß einem der vorstehenden Ansprüche zum Vorhersagen einer Immunreaktion gegen humane Leukozytenantigene (HLA) nach einer Transplantation, wobei das Verfahren Folgendes umfasst:
a. Bestimmen der Anzahl der vorhergesagten indirekt erkannten HLA-Epitope (PIRCHE), wobei die PIRCHE empfänger- oder spenderspezifische HLA-abgeleitete Peptide von dem nicht übereinstimmenden Empfänger-HLA-Allel sind und vorhergesagt wird, dass sie von einem gemeinsamen (übereinstimmenden) HLA-Molekül präsentiert werden, für die Auswahl eines Transplantationsspenders zum Bereitstellen von Transplantationsmaterial, wobei HLA-Allelgruppen-Typisierungsinformationen verfügbar sind und HLAproteinspezifische Typisierungsinformationen für den Spender nicht verfügbar sind, oder
b. Bestimmen der Anzahl der vorhergesagten indirekt erkannten HLA-Epitope (PIRCHE), wobei die PIRCHE empfänger- oder spenderspezifische HLA-abgeleitete Peptide von dem nicht übereinstimmenden Spender-HLA-Allel sind und vorhergesagt wird, dass sie von einem gemeinsamen (übereinstimmenden) und nicht gemeinsamen (nicht übereinstimmenden) HLA-Molekül präsentiert werden, für die Auswahl eines Transplantationsspenders zum Bereitstellen von Transplantationsmaterial, wobei die HLAproteinspezifische Typisierungsinformation für den Spender und den Empfänger nicht verfügbar ist.

15. System zum Auswählen eines Transplantationsspenders zum Bereitstellen von Transplantationsmaterial, wobei das System Rechenvorrichtungen, Datenspeichervorrichtungen und/oder Software als Systemkomponenten umfasst, wobei die Komponenten dazu konfiguriert sind, mit einer oder mehreren der anderen Komponenten zu interagieren und die computerimplementierten Schritte des Verfahrens gemäß einem der vorstehenden Ansprüche auszuführen.

## Revendications

1. Méthode de sélection d'un donneur pour fournir une matière de transplantation, comprenant :
a. la fourniture d'une représentation électronique de données de typage HLA d'un receveur de transplantation et d'un ou de plusieurs donneurs de transplantation candidats, dans laquelle un typage de protéine HLA spécifique à deux champs n'est pas disponible pour ledit ou lesdits donneurs et/ou receveur,
b. la détermination dans une méthode mise en œuvre par ordinateur du nombre d'épitopes HLA prédits reconnus indirectement (PIRCHES) (valeur PIRCHE) pour plusieurs protéines HLA spécifiques du groupe d'allèles donneurs du locus HLA qui sont appariés ou potentiellement non appariés entre ledit donneur et ledit receveur (groupe d'allèles donneurs non appariés) ; à l'aide des données fournies à l'étape (a) et
c. la sélection ou le rejet d'un ou de plusieurs desdits donneurs de transplantation candidats dans une méthode mise en œuvre par ordinateur selon les valeurs PIRCHE déterminées à l'étape b).

2. Méthode de sélection d'un groupe d'allèles non appariés d'un donneur de transplantation, comprenant :
a. la fourniture d'une représentation électronique de données de typage HLA d'un receveur de transplantation et d'un ou de plusieurs donneurs de transplantation candidats, dans laquelle un typage de protéine HLA spécifique à deux champs n'est pas disponible pour ledit ou lesdits donneurs et/ou receveur,
b. la détermination dans une méthode mise en œuvre par ordinateur du nombre d'épitopes HLA prédits reconnus indirectement (PIRCHES) (valeur PIRCHE) pour plusieurs protéines HLA spécifiques de plusieurs groupes d'allèles d'un allèle non apparié entre ledit donneur et ledit receveur ; à l'aide des données fournies à l'étape (a) et
c. la sélection ou le rejet d'un ou de plusieurs groupes d'allèles non appariés d'un donneur de transplantation dans une méthode mise en œuvre par ordinateur selon les valeurs PIRCHE déterminées à l'étape b).

3. Méthode selon la revendication 1, dans laquelle au moins un mésappariement HLA (au niveau de n'importe quel locus HLA) est présent entre ledit ou lesdits donneur(s) et ledit receveur au niveau du groupe d'allèles.

4. Méthode selon l'une quelconque des revendications précédentes, dans laquelle des informations de typage de protéine HLA spécifiques sont disponibles pour ledit receveur et des informations de typage de protéine HLA spécifiques ne sont pas disponibles pour ledit donneur, ou dans laquelle un typage de protéine HLA spécifique à deux champs n'est pas disponible et des informations de typage de groupe d'allèles HLA sont disponibles pour ledit receveur.

5. Méthode selon l'une quelconque des revendications précédentes, dans laquelle ledit donneur de transplantation ou groupe d'allèles non appariés d'un donneur de transplantation est associé à un risque réduit d'une réponse immunitaire indésirable contre les antigènes leucocytaires humains (HLA) après transplantation.

6. Méthode selon l'une quelconque des revendications précédentes, dans laquelle la valeur PIRCHE est la somme des valeurs PIRCHE-I et PIRCHE-II.

7. Méthode selon l'une quelconque des revendications précédentes, dans laquelle plus d'un donneur de transplantation candidat est fourni, et dans laquelle la probabilité (risque) d'une réponse immunitaire indésirable contre les antigènes leucocytaires humains (HLA) chez le receveur après la transplantation est plus faible pour la matière de transplantation obtenue à partir de donneurs avec une proportion relativement plus élevée de protéines HLA spécifiques du groupe d'allèles donneurs non appariés qui présentent des valeurs PIRCHE relativement inférieures à celles d'autres donneurs candidats, ou des valeurs PIRCHE inférieures ou égales à une valeur prédéfinie (définie par l'utilisateur), par rapport à d'autres donneurs candidats.

8. Méthode selon l'une quelconque des revendications précédentes, comprenant :
a. la détermination de la distribution de valeurs PIRCHE de protéines HLA spécifiques du groupe d'allèles donneurs non appariés, dans laquelle un donneur de transplantation candidat est sélectionné pour fournir une matière de transplantation lorsqu'une proportion relativement plus élevée de protéines HLA spécifiques du groupe d'allèles donneurs non appariés présentent des valeurs PIRCHE relativement inférieures à celles d'autres donneurs candidats,
b. la détermination de la valeur PIRCHE médiane des protéines HLA spécifiques du groupe d'allèles donneurs non appariés, dans laquelle un donneur de transplantation candidat est sélectionné pour fournir une matière de transplantation lorsque la valeur PIRCHE médiane est inférieure ou égale à une valeur prédéfinie, de préférence dans laquelle un donneur de transplantation candidat est sélectionné pour fournir une matière de transplantation lorsque la valeur PIRCHE médiane est inférieure ou égale à 10, plus préférablement inférieure ou égale à 5, et éventuellement la détermination de la valeur PIRCHE médiane et l'écart type des protéines HLA spécifiques du groupe d'allèles donneurs non appariés, ou
c. la détermination du pourcentage des protéines HLA spécifiques du groupe d'allèles donneurs non appariés qui ont une valeur PIRCHE inférieure ou égale à une valeur prédéfinie, dans laquelle un donneur de transplantation candidat est sélectionné pour fournir une matière de transplantation lorsque plus du pourcentage prédéfini des protéines HLA spécifiques du groupe d'allèles donneurs non appariés ont une valeur PIRCHE inférieure ou égale à une valeur prédéfinie, de préférence dans laquelle le pourcentage prédéfini est de 50 % et/ou la valeur prédéfinie est inférieure ou égale à 10, plus préférablement inférieure ou égale à 5.

9. Méthode selon l'une quelconque des revendications précédentes, dans laquelle le donneur de transplantation candidat est sélectionné pour fournir une matière de transplantation pour une procédure de transplantation biologique, dans laquelle la transplantation biologique est une transplantation d'organe ou une transplantation de cellules, de préférence une transplantation de cellules souches, telle qu'une transplantation de cellules souches hématopoïétiques (CSH), par exemple dans laquelle lesdites CSH sont obtenues à partir de sang de cordon.

10. Méthode selon l'une quelconque des revendications précédentes, dans laquelle une ou plusieurs étapes de la méthode sont exécutées dans une méthode mise en œuvre par ordinateur, dans laquelle
a. la fourniture d'un ou de donneurs de transplantation candidats et d'un receveur de transplantation comprend une représentation électronique (in silico) et/ou un traitement des informations de typage HLA disponibles pour ledit donneur et ledit receveur dans une méthode mise en œuvre par ordinateur,
b. les séquences de protéines HLA spécifiques de tout groupe d'allèles donneurs non appariés sont fournies, obtenues et/ou traitées comme une représentation électronique (in silico) des séquences de protéines HLA de n'importe quel locus HLA donné,
c. la représentation électronique (in silico) des séquences de protéines HLA de n'importe quel locus HLA donné est stockée dans une banque de données comprenant plusieurs séquences d'allèles HLA connues, et/ou
d. les donneurs candidats sont générés électroniquement (in silico) pour représenter toutes les combinaisons possibles de protéines HLA spécifiques non appariées avec le receveur de transplantation dans une méthode mise en œuvre par ordinateur.

11. Méthode selon l'une quelconque des revendications précédentes, comprenant la détermination de la valeur PIRCHE pour plusieurs protéines HLA spécifiques de plusieurs groupes d'allèles d'un allèle non apparié pour plusieurs loci HLA non appariés, identifiant ainsi un locus HLA non apparié associé à des valeurs PIRCHE relativement faibles par rapport à d'autres loci HLA.

12. Méthode selon l'une quelconque des revendications précédentes, comprenant le traitement d'informations représentant la fréquence de n'importe quelle protéine HLA spécifique donnée et/ou groupe d'allèles HLA (haplotype ou génotype) dans une ou plusieurs populations humaines, tel que défini par l'âge, le sexe et/ou l'ethnie, lors de la sélection d'un donneur ou d'un groupe d'allèles non appariés.

13. Méthode pour réduire le risque d'une réponse immunitaire indésirable contre les antigènes leucocytaires humains (HLA) après transplantation chez un receveur et/ou pour augmenter la probabilité d'obtenir un donneur de transplantation non apparié associé à un risque réduit d'induction de ladite réponse immunitaire indésirable comprenant la mise en œuvre de la méthode selon l'une quelconque des revendications précédentes.

14. Méthode selon l'une quelconque des revendications précédentes, pour prédire une réponse immunitaire contre les antigènes leucocytaires humains (HLA) après transplantation, ladite méthode comprenant :
a. la détermination du nombre d'épitopes HLA prédits reconnus indirectement (PIRCHE), dans laquelle lesdits PIRCHE sont des peptides dérivés de HLA spécifiques au receveur ou au donneur provenant de l'allèle HLA receveur non apparié et sont prédits comme étant présentés par une molécule HLA partagée (appariée), pour la sélection d'un donneur de transplantation pour fournir une matière de transplantation, dans laquelle des informations de typage de groupe d'allèles HLA sont disponibles et des informations de typage spécifiques de protéine HLA ne sont pas disponibles pour ledit donneur, ou
b. la détermination du nombre d'épitopes HLA prédits reconnus indirectement (PIRCHE), dans laquelle lesdits PIRCHE sont des peptides dérivés de HLA spécifiques au receveur ou au donneur provenant de l'allèle HLA donneur non apparié et sont prédits comme étant présentés par une molécule HLA partagée (appariée) et non partagée (non appariée), pour la sélection d'un donneur de transplantation pour fournir une matière de transplantation, dans laquelle des informations de typage spécifiques de protéine HLA ne sont pas disponibles pour ledit donneur et receveur.

15. Système de sélection d'un donneur de transplantation pour fournir une matière de transplantation, ledit système comprenant des dispositifs informatiques, des dispositifs de stockage de données et/ou un logiciel en tant que composants de système, dans lequel lesdits composants sont configurés pour interagir avec un ou plusieurs desdits autres composants et pour exécuter les étapes mises en œuvre par ordinateur de la méthode selon l'une quelconque des revendications précédentes.
